(19) Europäisches Patentamt
European Patent Office
Office européen des brevets

(11) EP 0 879 811 A1

(12) EUROPEAN PATENT APPLICATION

(43) Date of publication:
25.11.1998 Bulletin 1998/48

(51) Int Cl.[6]: C07C 50/38, G03G 5/06

(21) Application number: 98304106.2

(22) Date of filing: 22.05.1998

(84) Designated Contracting States:
AT BE CH CY DE DK ES FI FR GB GR IE IT LI LU MC NL PT SE
Designated Extension States:
AL LT LV MK RO SI

(30) Priority: 23.05.1997 JP 133595/97

(71) Applicant: MITA INDUSTRIAL CO. LTD.
Chuo-ku , Osaka 540 (JP)

(72) Inventors:
- Okada, Hideki
  Tamatsukuri, Chuo-ku, Osaka 540 (JP)
- Hamasaki, Kazunari
  Tamatsukuri, Chuo-ku, Osaka 540 (JP)

(74) Representative: Cresswell, Thomas Anthony
J.A. KEMP & CO.
14 South Square
Gray's Inn
London WC1R 5LX (GB)

# (54) Naphthoquinone derivatives and electro-photosensitive material using same

(57) A novel dinaphthoquinone derivative suited for use as an electron-transporting agent, and a highly sensitive electro-photosensitive material using the same dinaphthoquinone derivative. The naphthoquinone derivative is represented by the general formula (1),

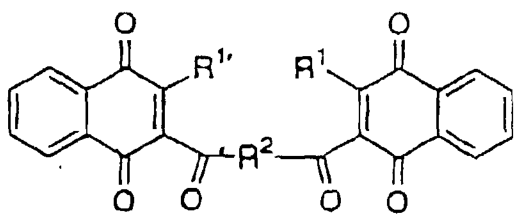

(1)

wherein $R^1$ is an aryl group that may have a substituent, and $R^2$ is an alkylene group that may have a substituent or an arylene group that may have a substituent.

The derivative is contained, as an electron-transporting agent, in the photosensitive layer of the electro-photosensitive material.

EP 0 879 811 A1

FIG.1

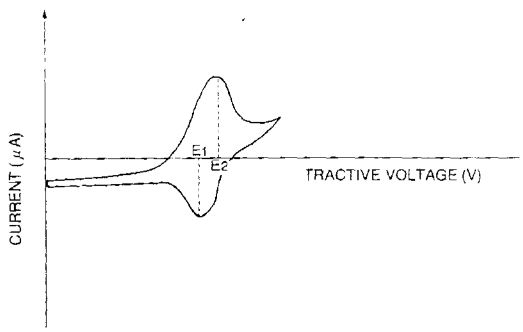

## Description

### BACKGROUND OF THE INVENTION

1. Field of the Invention

The present invention relates to novel naphthoquinone derivatives and to an electro-photosensitive material used for image-forming apparatuses such as electrostatic copying machine, facsimile, laser-beam printer, etc.

2. Description of the Prior Art

Electro-photosensitive materials comprising various materials have been used in the image-forming apparatuses that employ Carlson's process, such as copying machine, facsimile, laser printer, etc. One of them is an inorganic photosensitive material using an inorganic material such as selenium as a photosensitive layer, and another one is an organic photosensitive material (OPC) using an organic material as a photosensitive layer. Among them, the organic photosensitive material offers many advantages in regard to that it is produced cheaply and efficiently compared with the inorganic photosensitive material and, besides, it is harmless. Therefore, study has been extensively forwarded concerning the organic photosensitive material.

Many of the organic photosensitive materials pertain to those of the laminated-layer type in which a charge-generating layer and a charge-transporting layer are laminated one upon the other, i.e., pertain to those of the function separation type, but there has also been known the one of the so-called single-layer type in which a charge-generating agent and a charge-transporting agent are dispersed in a single photosensitive layer.

The charge-transporting agent used for these photosensitive materials must have a high carrier mobility. Most of the charge-transporting agents featuring a high carrier mobility have a positive hole-transporting property. From the standpoint of mechanical strength, therefore, the organic photosensitive materials put into practical use are limited to those of the negatively-charged laminated-layer type having a charge-transporting layer as the outermost layer. However, the negatively-charged organic photosensitive material utilizes corona discharge of negative polarity arousing problems such as generating ozone in large amounts, contaminating the environment, and deteriorating the photosensitive material.

In order to preclude such defects, therefore, study has been forwarded to use an electron-transporting agent as the charge-transporting agent. For instance, Japanese Laid-Open Patent Publication No. 206349/1989 proposes the use of a compound having a diphenoquinone structure or a benzoquinone structure as an electron-transporting agent for the electro-photosensitive material. Furthermore, Japanese Laid-Open Patent Publication No. 110227/1994 proposes the use of a compound having a benzoquinone structure or a naphthoquinone structure as an electron-transporting agent for the electro-photosensitive material.

However, the conventional electron-transporting agents such as diphenoquinone derivatives, benzoquinone derivatives, and naphthoquinone derivatives, cannot be matched with the charge-generating agent, and permit electrons to be injected in insufficient amounts from the charge-generating agent into the electron-transporting agent. Besides, such electron-transporting agents are poorly compatible with the binder resin, and cause the hopping distance to be lengthened, permitting the electrons to move poorly in a low electric field. Therefore, the photosensitive materials containing the conventional electron-transporting agents exhibit a high residual potential and a low sensitivity as will become obvious from the testing of sensitivity described in Examples appearing later.

As described above, many of the organic photosensitive materials that have now been put irco practical use are equipped with a photosensitive layer of the laminated-layer type. In contrast, the photosensitive material equipped with a photosensitive layer of the single-layer type offers many advantages in that it is simple in the structure, is easily prepared, suppresses the occurrence of defect in the film, and exhibits improved optical properties.

Besides, the photosensitive material equippec with the photosensitive layer of the single-layer type uses the electron-transporting agent and the positive hole-transporting agent in combination as a charge-transporting agent. In other words, the photosensitive material can be used either as the one of the positively charged type or the one of the negatively charged type, enabling the range of application to be expanded. However, the conventional electron-transporting agent interacts with the positive hole-transporting agent to impair the transportation of electrons and positive holes. Because of this reason, the photosensitive material equipped with a photosensitive layer of the single-layer type has not been put into wide use.

### SUMMARY OF THE INVENTION

The object of the present invention is to provide a novel compound which can be favorably used as an electron-transporting agent in the electro-photosensitive material, and an electro-photosensitive material using this novel compound featuring higher sensitivity than the conventional photosensitive materials, by solving the above-mentioned technical problems.

In order to solve the above-mentioned problems, the present invention provides a naphthoquinone derivative represented by the general formula (1),

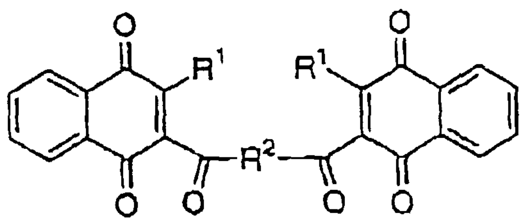

(1)

wherein $R^1$ is an aryl group which may have a substituent, and $R^2$ is an alkylene group which may have a substituent, an arylene group which may have a substituent, a group (i),

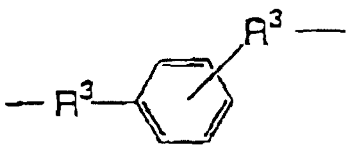

(i)

wherein $R^3$ is an alkylene group which may have a substituent,
or a group (ii),

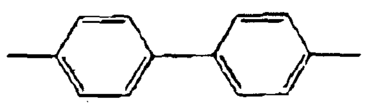

(ii)

The naphthoquinone derivative of the present invention expressed by the above-mentioned general formula (1) exhibits excellent electron accepting property owing to the action of a group >C=O of a naphthoquinone ring, exhibits favorable solubility in a solvent and compatibility with a binder resin owing to the action of the group $R^1$, and is, hence, homogeneously dispersed in the photosensitive layer, featuring short hopping distance among the electrons and, particularly, exhibiting excellent electron transporting property in a low electric field and excellent macching with the charge-generating agent. By using the derivative (1) as an electron-transporting agent for the electro-photosensitive material, therefore, it is allowed to provide a highly sensitive photosensitive material.

In particular, the naphthoquinone derivative (1) of the present invention is a combination of two naphthoquinone rings that feature excellent electron-transporting property and electron-accepting property owing to the actions of the above-mentioned substituents, and is estimated to exhibit further improved electron-transporting property.

By utilizing a high electron-transporting ability, furthermore, the naphthoquinone derivative (1) of the present invention can be used for such applications as solar cells and EL elements.

The electro-photosensitive material of the present invention has a photosensitive layer formed on an electrically conducting substrate, and a feature resides in that the photosensitive layer contains a naphthoquinone derivative represented by the general formula (1).

The photosensitive layer containing the naphthoquinone derivative (1) exhibits excellent electron-transporting property in a low electric field, permits the electrons and the positive holes to recombine together at a decreased ratio in the layer, permits the apparent charge-generating efficiency to approach a practical value, and, hence, exhibits improved sensitivity. Moreover, the photosensitive material exhibits a low residual potential, and improved stability and durability even when it is repetitively exposed to light.

In particular, the naphthoquinone derivative (1) does not develop interaction relative to the positive hole-transporting agent, and does not impair the transportation of electrons and positive holes. Particularly, when the naphthoquinone derivative (1) is used for the photosensitive layer of the single-layer type which also contains a positive-hole transporting agent, there is obtained a more sensitive photosensitive material.

The sensitivity of the photosensitive material is further improved when the photosensitive layer contains an electron-transporting agent which has an oxidation-reduction potential of -0.8 to -1.4 V.

This is because, it is estimated that the electron-transporting agent works to extract electrons from the charge-generating agent and transmits them to the naphthoquinone derivative (1), enabling the electrons to be smoothly injected into the naphthoquinone derivative (1) from the charge-generating agent.

By taking compatibility with the naphthoquinone derivative (1) into consideration, furthermore, it is desired to use, as the electron-transporting agent, a diphenoquinone derivative represented by the general formula (2),

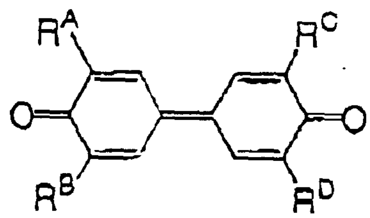

(2)

wherein $R^A$, $R^B$, $R^C$ and $R^D$ may be the same or different, and are hydrogen atoms, alkyl groups, alkoxyl groups, aryl groups, aralkyl groups, cycloalkyl groups or amino groups,
or a benzoquinone derivative represented by the general formula (3).

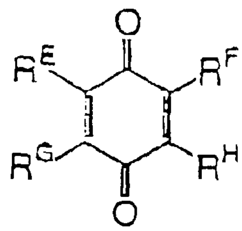

(3)

wherein $R^E$, $R^F$, $R^G$ and $R^H$ may be the same or different, and are hydrogen atoms, alkyl groups, alkoxyl groups, aryl groups, aralkyl groups, cycloalkyl groups or amino groups which may have a substituent.

## BRIEF DESCRIPTION OF THE DRAWINGS

Fig. 1 is a graph illustrating a relationship between a current (A) and a tractive voltage (V) for finding an oxidation-reduction potential according to the present invention;
Fig. 2 is a graph illustrating a 1H-NMR spectrum of a naphthoquinone derivative (1-1) of the present invention;
Fig. 3 is a graph illustrating an IR spectrum of the naphthoquinone derivative (1-1) of the present invention;
Fig. 4 is a graph illustrating an MS spectrum of the naphthoquinone derivative (1-1) of the present invention;
Fig. 5 is a graph illustrating a 1H-NMR spectrum of a naphthoquinone derivative (1-2) of the present invention;
Fig. 6 is a graph illustrating an IR spectrum of the naphthoquinone derivative (1-2) of the present invention;
Fig. 7 is a graph illustrating a 1H-NMR spectrum of a naphthoquinone derivative (1-3) of the present invention;
Fig. 8 is a graph illustrating an IR spectrum of the naphthoquinone derivative (1-3) of the present invention;
Fig. 9 is a graph illustrating a 1H-NMR spectrum of a naphthoquinone derivative (1-4) of the present invention;
Fig. 10 is a graph illustrating an IR spectrum of the naphthoquinone derivative (1-4) of the present invention; and
Fig. 11 is a graph illustrating an MS spectrum of the naphthoquinone derivative (1-4) of the present invention.

## DETAILED DESCRIPTION OF THE INVENTION

The naphthoquinone derivative (1) used for the electro-photosensitive material of the present invention will, first, be described.

In the naphthoquinone derivative (1) represented by the general formula (1), examples of the aryl group denoted by $R^1$ include phenyl, tolyl, xylyl, biphenylyl, o-terphenyl, naphthyl, anthryl and phenanthryl. In general, the aryl group preferably comprises 1-4 rings either as a fused system or as ring assemblies, one or more of the rings having one or more (preferably one, two or three) alkyl substituents, each preferably having 1-3 carbon atoms.

Examples of the arylene group denoted by $R^2$ include phenylene, naphthylene, anthracenediyl and phenanthrylene.

Examples of the alkylene group denoted by $R^2$ or $R^3$ include those having 1 to 6 carbon atoms, such as methylene,

ethylene, trimethylene, tetramethylene, pentamethylene and hexamethylene.

The alkylene group may have a substituent which is an alkyl group or a halogen atom.

The aryl group may have a substituent which is an alkyl group, aralkyl group, alkoxyl group, alkanoyl group, halogen atom, aryl group or alkoxycarbonyl group.

The alkyl group will be the one having 1 to 6 carbon atoms, such as methyl, ethyl, n-propyl, isopropyl, n-butyl, isobutyl, s-butyl, t-butyl, pentyl or hexyl.

Examples of the halogen atom include fluorine, chlorine, bromine and iodine. Examples of the alkoxyl group include those having 1 to 6 carbon atoms, such as methoxy, ethoxy, propoxy, isopropoxy, butoxy, isobutoxy, s-butoxy, t-butoxy, pentyloxy, and hexyloxy.

Examples of the aralkyl group include those having 1 to 6 carbon atoms of the alkyl portion, such as benzyl, 1-phenylethyl, 3-phenylpropyl, 4-phenylbutyl, 5-phenylpentyl and 6-phenylhexyl.

Examples of the alkoxycarbonyl include those having 1 to 6 carbon atoms of the alkoxy portion, such as, methoxycarbonyl, ethoxycarbonyl, propoxycarbonyl, isopropoxycarbonyl, butoxycarbonyl, t-butoxycarbonyl, pentyloxycarbonyl and hexyloxycarbonyl.

Examples of the alkanoyl group include those having 1 to 6 carbon atoms, such as acetyl, propionyl, butylyl, isobutylyl, pentanoyl, t-butylcarbonyl and hexanoyl.

Examples of the aryl group include those described above.

The alkyl group and the aryl group may have a substituent. The above-mentioned halogen atom can be exemplified as a substituent for the alkyl group. Moreover, the above-mentioned alkyl group or the halogen atom can be exemplified as a substituent for the aryl group.

The naphthoquinone derivative in which the group R2 is an alkylene group exhibits particularly excellent electron-transporting ability making it possible to obtain a highly sensitive photosensitive material.

The naphthoquinone derivative in which the group R2 is the group (i), the group (ii) or arylene group, exhibits a high glass transition temperature Tg and makes it possible to increase Tg of the photosensitive layer and, hence, prevents the surface of the photosensitive material from scarred by a cleaning blade that is press-contacted to the surface of the photosensitive material when the device is halted. Moreover, the naphthoquinone derivative in which the arylene group has an alkyl group as a substituent exhibits excellent solubility in a solvent and compatibility with the binder resin.

Concrete examples of the naphthoquinone derivative (1) represented by the general formula (1) include the following compounds represented by the formulas (1-1) to (1-9).

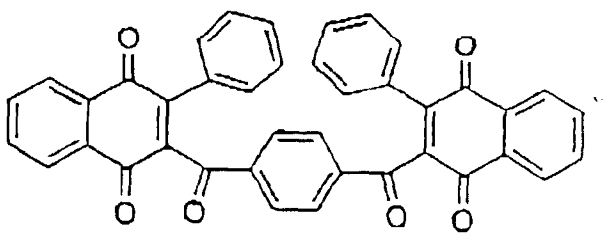

(1-1)

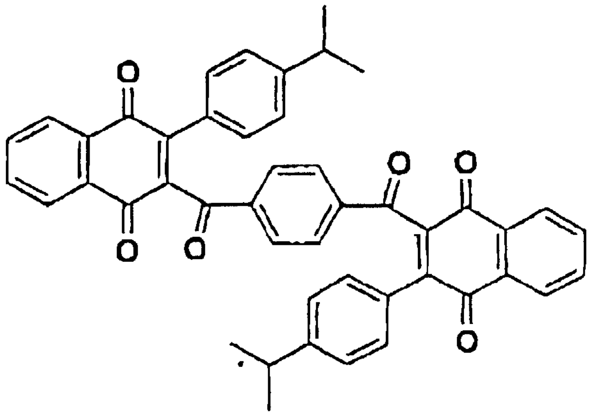

(1-2)

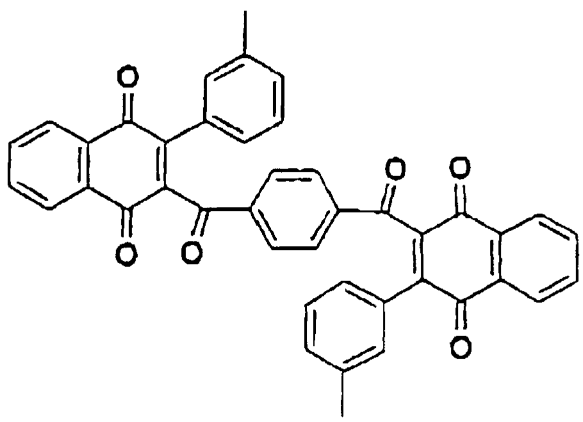

(1-3)

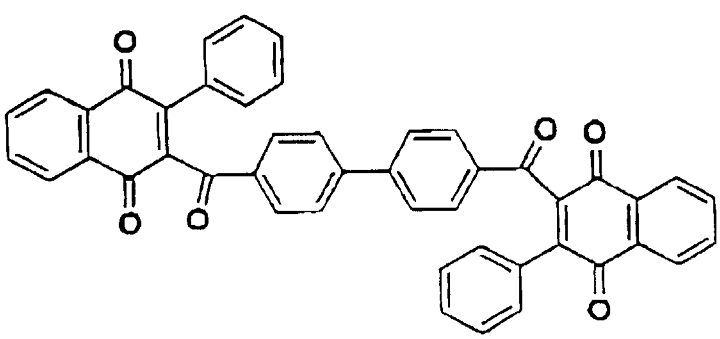

(1-4)

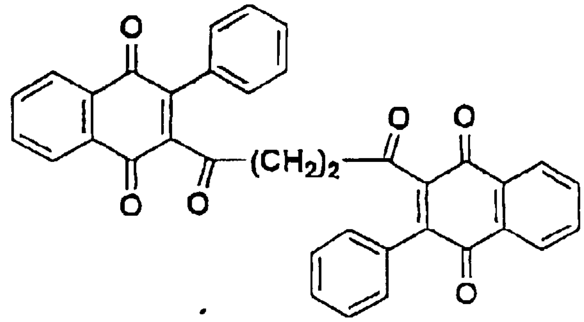

(1-5)

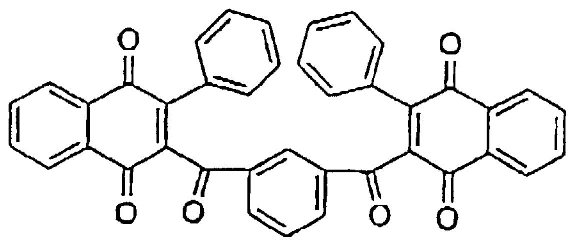

(1-6)

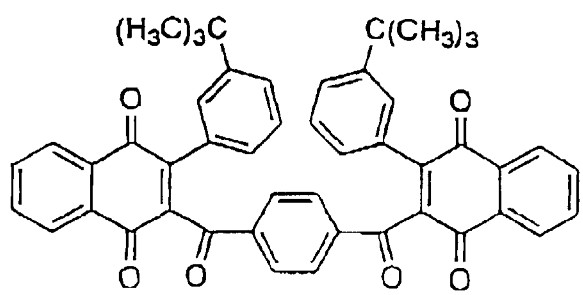

(1-7)

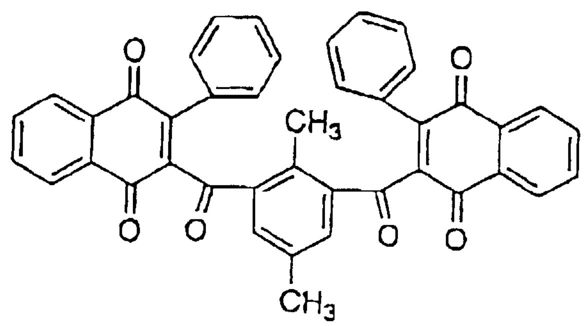

(1-8)

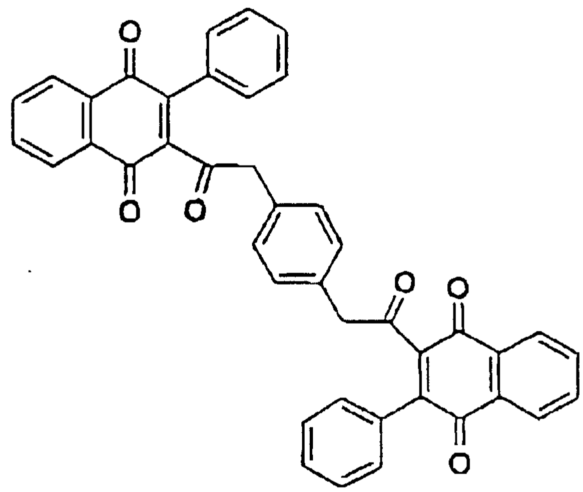

(1-9)

The naphthoquinone derivative represented by the general formula (1) is synthesized as expressed by the following reaction formulas I and II.

Reaction formula I.

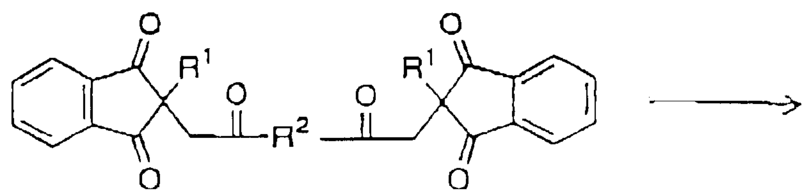

(4)

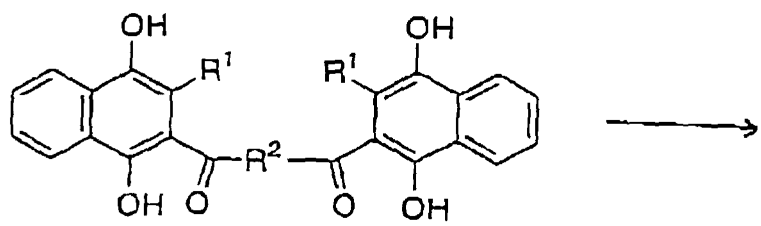

(5)

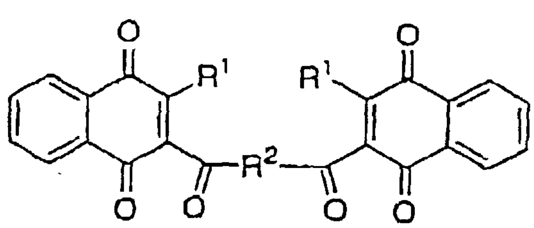

(1)

wherein $R^1$ and $R^2$ are as defined above.

As shown in this reaction, the naphthoquinone derivative (1) of the present invention is obtained by isomerizing a 1,3-indandione derivative represented by the general formula (4) without solvent or in a suitable solvent in the presence of a sodium hydride thereby to obtain a 1,4-dihydroxynaphthalene derivative represented by the general formula (5), followed by oxidation.

It is desired that sodium hydride is used at least in an equivalent amount and, preferably, in an amount of about 1.2 mol times as great with respect to the compound (4).

The reaction is carried out, usually, at 40 to 100°C and, preferably, at 70 to 80°C, and ends in about four hours.

Reaction formula II.

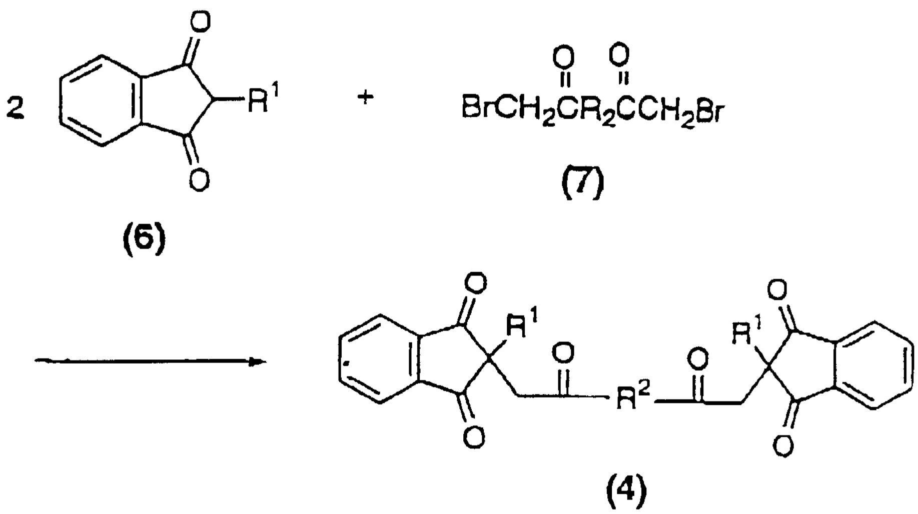

(6) (7) (4)

wherein $R^1$ and $R^2$ are as defined above.

This reaction is to obtain the compound of the above-mentioned formula (4) which is the starting material for the above-mentioned reaction formula I by reacting a 1,3-indandione derivative represented by the general formula (6) with a dibromoacetyl derivative presented by the general formula (7) in the presence of a base without solvent or in a suitable solvent.

Examples of the base include sodium alkoxide such as sodium methoxide, and a metal hydride such as sodium hydride. It is desired that the base is used at least in an equivalent amount and, preferably, in an amount of about 1.2 mol times as great with respect to the compound (6).

The reaction is carried out, usually, at 40 to 100°C and, preferably, at 70 to 80°C, and ends in about five hours.

As the solvent, there can be used, for example, benzene, methylene chloride, chloroform, tetrahydrofuran, dimethylformamide or dimethyl sulfoxide.

Next, the electro-photosensitive material of the present invention will be described in detail.

As described above, the electro-photosensitive material of the present invention is provided with a photosensitive layer containing the naphthoquinone derivative (1) represented by the general formula (1) on an electrically conducting substrate.

The electro-photosensitive material of the present invention may be either of the single-layer type or the laminated-layer type, but the effect of the electron-transporting agent appears conspicuously in the single-layer type photosensitive material.

The electro-photosensitive material of the single-layer type has a single photosensitive layer formed on the electrically conducting substrate. This photosensitive layer comprises a binder resin containing, at least, a charge-generating agent, a positive hole-transporting agent, as well as a naphthoquinone derivative (1) as an electron-transporting agent.

The single-layer type photosensitive material can be used being either positively charged or negatively charged but is desirably used being positively charged.

On the other hand, the laminated-layer type electro-photosensitive material has at least a charge-generating layer and a charge-transporting layer formed in this order on the electrically conducting substrate, and wherein the naphthoquinone derivative (1) is contained as an electron-transporting agent in the charge-transporting layer. The laminated-layer type electro-photosensitive material has a greatly decreased residual potential compared with the conventional laminated-layer type electro-photosensitive materials, and exhibits improved sensitivity. It is further desired that the naphthoquinone derivative (1) is contained in the charge-generating layer, too, so that the electrons can be smoothly transferred from the charge-generating layer into the charge-transporting layer.

As described earlier, the naphthoquinone derivative (1) used for the electro-photosensitive material of the present invention exhibits good solubility in a solvent and compatibility with a binder resin, as well as excellent matching with the charge-generating agent, enabling the electrons to be smoothly injected and, particularly, exhibiting excellent electron-transporting property in a low electric field.

Therefore, in the single-layer type photosensitive material that is, for example, positively charged, the electrons emitted from the charge-generating agent are smoothly injected into the electron-transporting agent comprising a naphthoquinone derivative (I) represented by the general formula (1) in a step of exposure to light. Then, the electrons are exchanged in the electron-transporting agent and migrate onto the surface of the photosensitive layer thereby to cancel a positive charge (+) with which the surface of the photosensitive layer has been charged. On the other hand, the positive holes (+) are injected into the positive hole-transporting agent and migrate onto the surface of the electrically conducting substrate without being trapped on the way and cancel the negative charge (-) on the surface of the electrically conducting substrate. It is, thus, considered that the positively charged single-layer type photosensitive material exhibits improved sensitivity. The negatively charged single-layer type photosensitive layer permits the electric charge to migrate in simply the opposite direction and similarly exhibits improved sensitivity.

In the positively charged laminated-layer type photosensitive material, the electrons emitted from the charge-generating agent in the charge-generating layer are smoothly injected into the electron-transporting agent comprising a naphthoquinone derivative represented by the above-mentioned general formula (1) in the step of exposure to light. Then, the electrons are exchanged in the electron-transporting agent and migrate in the charge-transporting layer to reach the surface of the photosensitive layer thereby to cancel a positive charge (+) with which the surface of the photosensitive layer is charged. On the other hand, the positive holes (+) migrate from the charge-generating layer directly onto the surface of the electrically conducting substrate to thereby cancel the negative charge (-) on the surface of the electrically conducting substrate. It is, thus, considered that the positively charged laminated-layer type photosensitive material exhibits improved sensitivity.

The charge-generating agent that has absorbed light as a result of exposing the photosensitive material to light forms ion pairs [positive holes (+) and electrons (-)]. In order that the thus formed ion pairs work as free carriers to effectively cancel the electric charge on the surface, it is desired that the ion pairs are recombined and are extinguished at a small rate.

The electro-photosensitive material of the present invention may contain other electron-transporting agents together with the above-mentioned naphthoquinone derivative (1). In particular, an electron-transporting agent having an oxidation-reduction potential of from -0.8 to -1.4 V is preferably used. The reasons are as described below.

When an electron-transporting agent having an oxidation-reduction potential which is smaller than -1.4 V is used, the energy level of LUMO (which stands for an orbit of the lowest energy level among the molecular orbits without electron, and an excited electron usually migrates onto this orbit) becomes higher than that of the charge-generating agent. At the time when the ion pairs are formed, therefore, the electrons do not migrate into the electron-transporting agent, and the charge-generating efficiency is not improved.

On the other hand, when an electron-transporting agent having an oxidation-reduction potential which is greater than -1.4 V is used, the energy level of LUMO is lower than that of the charge-generating agent. At the time when the ion pairs are formed, therefore, the electrons migrate into the electron-transporting agent, and the ion pairs are easily separated into the carrier. That is, the electron-transporting agent acts upon generating the electric charge so that the generation efficiency is improved.

In order to maintain high sensitivity, on the other hand, the carrier traps should not be generated by the impurities when the free carriers are being migrated.

When the free carriers migrate, usually, there exist traps due to impurities of small amounts. The free carriers migrate while repeating trapping - out-from-trapping. When an electron transporting agent having an oxidation-reduction potential which is larger than -0.8 V is used, the separated free carriers are caused to drop onto a level on which out-from trapping is impossible; i.e., the carrier traps are formed, and the free carriers no longer migrate.

On the other hand, when an electron-transporting agent having an oxidation-reduction potential which is smaller than -0.8 V is used, no carrier trap is formed, and the free carriers easily migrate.

As shown in Fig. 1, the above-mentioned oxidation-reduction potential is calculated from the following formula by finding E1 and E2 that are shown in Fig. 1 from a relationship between the tractive voltage (v) and the current (μA).

$$\text{Oxidation-reduction potential (V)} = (E1 + E2)/2$$

The tractive voltage (V) and the current (μA) were measured based on a three-electrode type cyclic voltametry by using a measuring solution prepared from the following materials.

Electrodes: acting electrode (glassy carbon electrode), opposing electrode (platinum electrode).
Reference electrode: Silver-Nitric Acid Electrode (0.1 mol/liter $AgNO_3$ - acetonitrile solution)
Measuring solution:

Electrolyte: 0.1 mol of tetra-n-butylammonium perchlorate
Measuring substance: 0.001 mol of electron-transporting agent
Solvent: one liter of $CH_2Cl_2$

From the above-mentioned materials is prepared a solution for measurement.

There is no particular limitation on the electron-transporting agent provided its oxidation-reduction potential is from -0.8 to -1.4 V. There can be used, for example, a benzoquinone compound, a naphthoquinone compound, an anthraquinone compound, a diphenoquinone compound, a malonitrile compound, a thiopyran compound, a 2,4,8-trinitrothioxanthone, a fluorenone compound such as 3,4,5,7-tetranitro-9-fluorenone, a dinitroanthracene, a dinitroacridine, a nitroanthraquinone, and a dinitroanthraquinone.

When the compatibility with the charge-generating agent or with the naphthoquinone derivative (1) of the present invention is taken into consideration, however, it is most desired to use a compound which belongs to the diphenoquinone compound represented by the above-mentioned general formula (2) or which belongs to the benzoquinone compound represented by the above-mentioned general formula (3) and has an oxidation-reduction potential that lies within the above-mentioned range among the above-mentioned compounds.

Though there is no particular limitation, it is desired that two or more of $R^A$, $R^B$, $R^C$ and $R^D$ in the above-mentioned formulas are the same groups.

As the alkyl group, aralkyl group, alkoxyl group and aryl group in the above-mentioned formulas, these can be exemplified those groups same as the above-mentioned groups.

As the cycloalkyl group, there can be used the one having 3 to 6 carbon atoms such as cyclopropyl, cyclobutyl, cyclopentyl or cyclohexyl.

As the amino group that may have a substituent, there can be exemplified monomethylamino, dimethylamino, monoethylamino and diethylamino, in addition to amino.

Though there is no particular limitation, concrete examples of the diphenoquinone compound include a 3,5-dimethyl-3',5'-di(t-butyl)-4,4'-diphenoquinone (oxidation-reduction potential of -0.86 V) represented by the formula (2-1) and a 3,5,3',5'-tetrakis(t-butyl)-4,4'-diphenoquinone (oxidation-reduction potential of -0.94 V) represented by the formula (2-2).

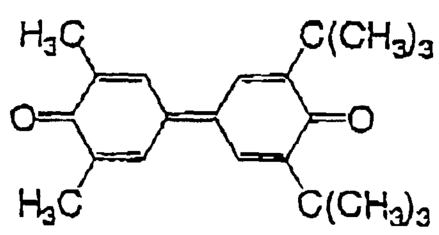

(2-1)

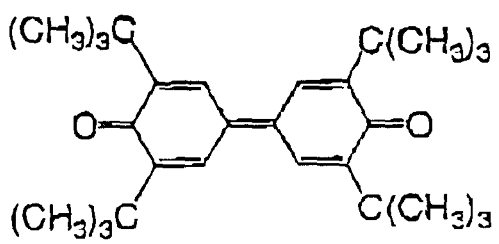

(2-2)

Though there is no particular limitation, concrete examples of the benzoquinone compound include a p-benzoquinone (oxidation-reduction potential of -0.81 V) represented by the formula (3-1) and a 2,6-di(t-butyl)-p-benzoquinone (oxidation-reduction potential of -1.31 V) represented by the formula (3-2).

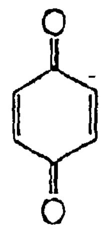

(3-1)

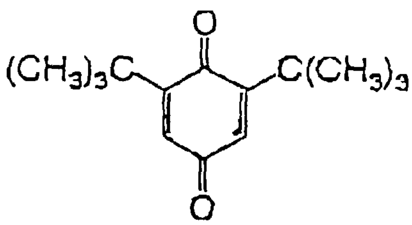

(3-2)

These electron-transporting agents can be used in a single kind or in a combination of two or more kinds.

In the present invention, the photosensitive layer may contain any other widely known electron-transporting agents in addition to the above-mentioned electron-transporting agents. For example, there can be raised the compounds represented by the following general formulas (ET1) to (ET16).

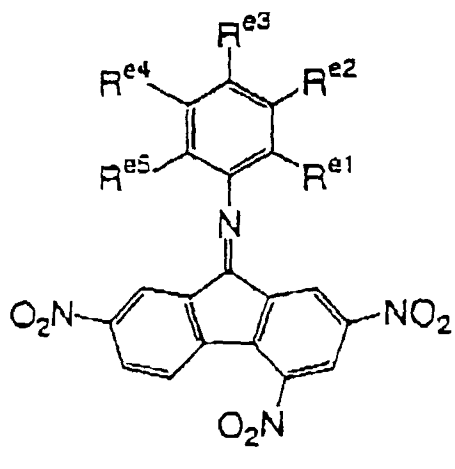

( ET1 )

wherein $R^{e1}$, $R^{e2}$, $R^{e3}$, $R^{e4}$ and $R^{e5}$ may be the same or different, and are hydrogen atoms, alkyl groups that may have a substituent, alkoxyl groups that may have a substituent, aryl groups that may have a substituent, aralkyl groups that may have a substituent, phenoxy groups that may have a substituent, or halogen atoms.

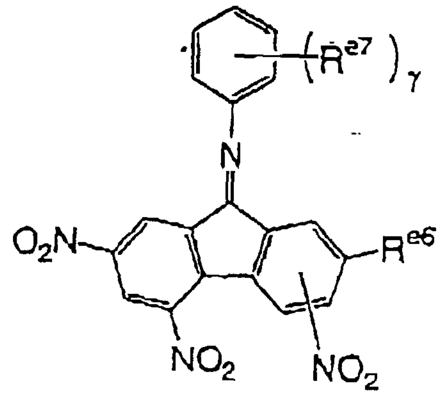

( ET2 )

wherein $R^{e6}$ is an alkyl group, $R^{e7}$ is an alkyl group that may have a substituent, an alkoxyl group that may have a substituent, an aryl group that may have a substituent, an aralkyl group that may have a substituent, a halogen atom, or a halogenated alkyl group, and $\gamma$ is an integer of from 0 to 5, but when $\gamma$ is 2 or larger, $R^{e7}$ may not be the same.

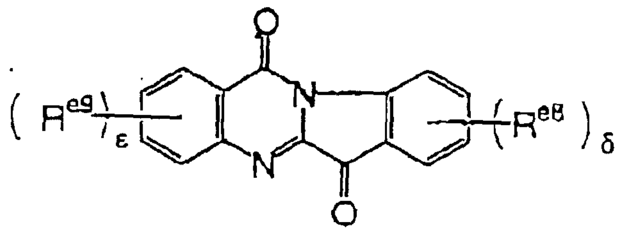

( ET3 )

wherein $R^{e8}$ and $R^{e9}$ may be the same or different, and are alkyl groups, $\delta$ is an integer of from 1 to 4, and $\varepsilon$ is an integer of from 0 to 4, but when $\delta$ and $\varepsilon$ are 2 or larger, $R^{e8}$ and $R^{e9}$ may not be the same,

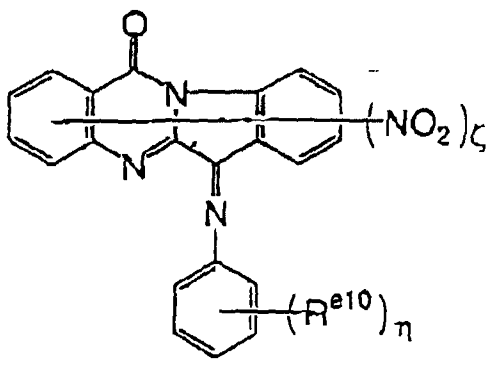

( ET4 )

wherein $R^{e10}$ is an alkyl group, an aryl group, an aralkyl group, an alkoxyl group, a halogenated alkyl group or a halogen atom, $\zeta$ is from 0 to 4, $\eta$ is an integer of from 0 to 5, but when $\eta$ is 2 or larger, $R^{e10}$ may not be the same.

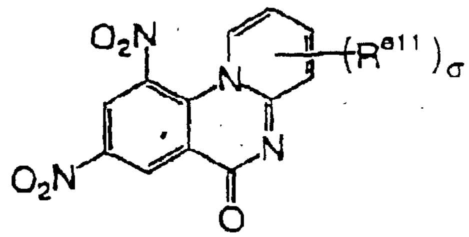

( ET5 )

wherein $R^{e11}$ is an alkyl group and $\sigma$ is an integer of from 1 to 4, but when $\sigma$ is 2 or larger, $R^{e11}$ may not be the same.

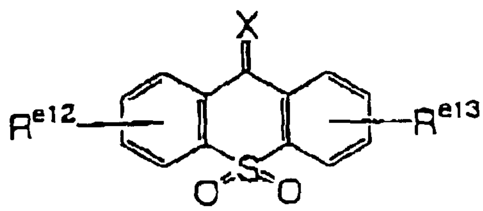

( ET6 )

wherein $R^{e12}$ and $R^{e13}$ may be the same or different, and are hydrogen atoms, halogen atoms, alkyl groups, aryl groups, aralkyloxycarbonyl groups, alkoxyl groups, hydroxyl groups, nitro groups or cyano groups, and X is an oxygen atom, a group =N-CN or a group $=C(CN)_2$.

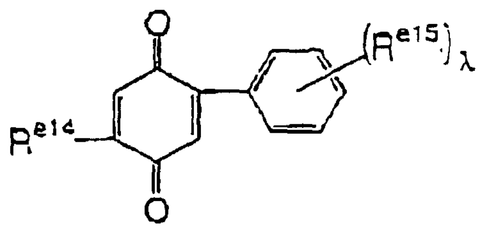

( ET7 )

wherein $R^{e14}$ is a hydrogen atom, a halogen atom, an alkyl group or a phenyl group that may have a substituent, $R^{e15}$ is a halogen atom, an alkyl group that may have a substituent, a phenyl group that may have a substituent, an alkoxycarbonyl group, an N-alkylcarbamoyl group, a cyano group or a nitro group, $\lambda$ is an integer of from 0 to 3, but when $\lambda$ is 2 or larger, $R^{e15}$ may not be the same.

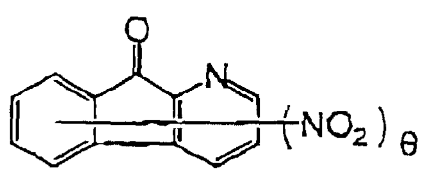

( ET8)

wherein $\theta$ is an integer or 1 or 2.

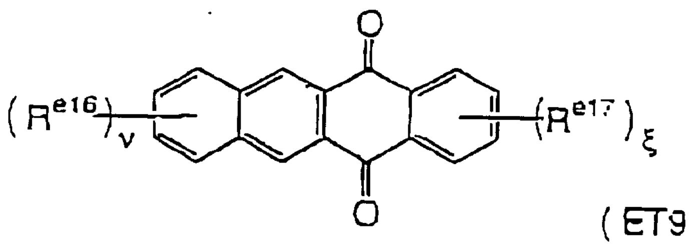

( ET9 )

wherein $R^{e16}$ and $R^{e17}$ may be the same or different, and are halogen atoms, alkyl groups that may have *a* substituent, cyano groups, nitro groups or alkoxylcarbonyl groups, $\nu$ and $\xi$ are integers of from 0 to 3 but when $\nu$ or $\xi$ are 2 or larger, $R^{e16}$ and $R^{e17}$ may not be the same.

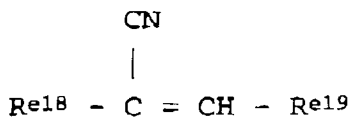

(ET10)

wherein $R^{e16}$ and $R^{e19}$ may be the same or different, and are phenyl groups, polycyclic aromatic groups or heterocyclic groups which may have a substituent.

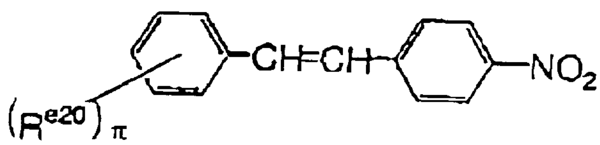

( ET11 )

wherein $R^{e20}$ is an amino group, a dialkylamino group, an alkoxyl group, an alkyl group or a phenyl group, $\pi$ is an integer of 1 or 2, but when $\pi$ is 2, $R^{e20}$ may not be the same.

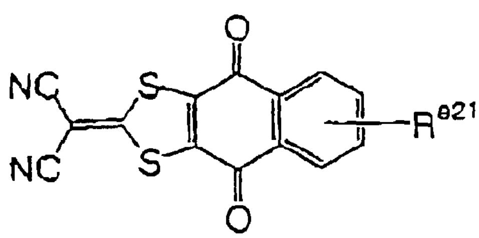

( ET12 )

wherein $R^{e21}$ is a hydrogen atom, an alkyl group, an aryl group, an alkoxyl group or an aralkyl group.

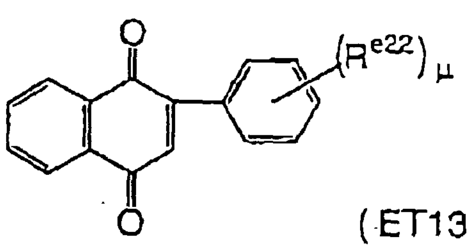

( ET13 )

wherein $R^{e22}$ is a halogen atom, an alkyl group that may have a substituent, a phenyl group that may have a

substituent, an alkoxycarbonyl group, an N-alkylcarbamoyl group, a cyano group or a nitro group, μ is an integer of from 0 to 3, but when μ is 2 or larger, $R^{e22}$ may not be the same.

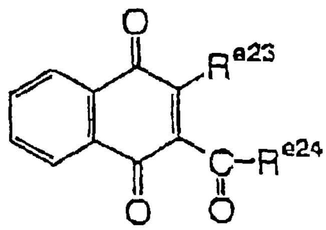

(ET14)

wherein $R^{e23}$ is an alkyl group that may have a substituent or an aryl group that may have a substituent, and $R^{e24}$ is an alkyl group that may have a substituent, an aryl group that may have a substituent, or a group $-O-R^{e24a}$, wherein $R^{e24a}$ is an alkyl group that may have a substituent or an aryl group that may have a substituent.

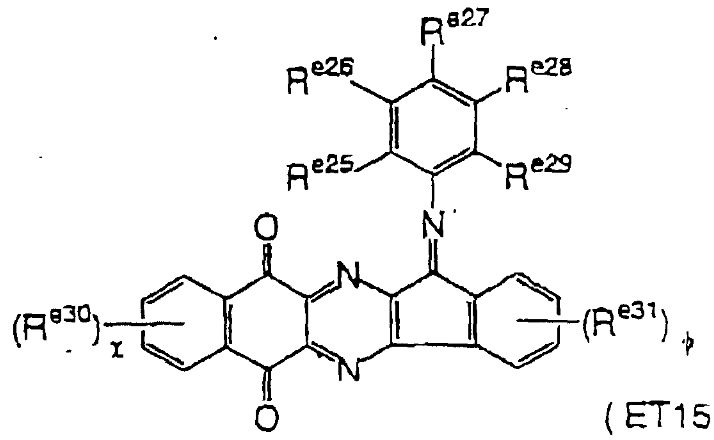

(ET15)

wherein $R^{e25}$, $R^{e26}$, $R^{e27}$, $R^{e28}$, $R^{e29}$, $R^{e30}$ and $R^{e31}$ may be the same or different, and are alkyl groups, aryl groups, aralkyl groups, alkoxyl groups, halogen atoms or halogenated alkyl groups, χ and φ may be the same or different and are integers of from 0 to 4.

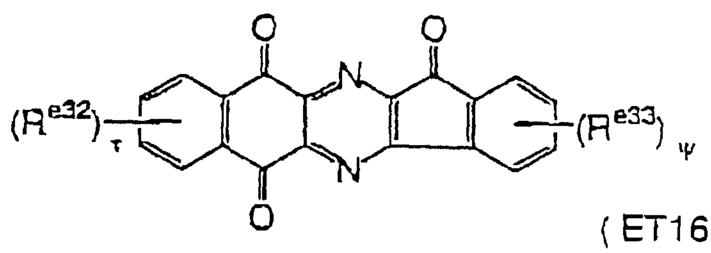

(ET16)

wherein $R^{e32}$ and $R^{e33}$ may be the same or different, and are alkyl groups, aryl groups, alkoxyl groups, halogen atoms or halogenated alkyl groups, τ and ψ may be the same or different and are integers of from 0 to 4.

In the above-mentioned electron-transporting agents, the alkyl group, aryl group, alkoxycarbonyl group, aralkyl group, alkoxyl group, halogen atom and cycloalkyl group may be the same as those mentioned above.

As the heterocyclic group, there can be exemplified thienyl, furyl, pyrrolyl, pyrrolidinyl, oxazolyl, isooxazolyl, thiazolyl, isothiazolyl, imidazolyl, 2H-imidazolyl, pyrazolyl, triazolyl, tetrazolyl, pyranyl, pyridyl, piperidyl, piperidino, 3-morpholinyl, morpholino and thiazolyl. The heterocyclic group may also be condensed with an aromatic ring.

As the halogenated alkyl group, there can be exemplified those having 1 to 6 carbon atoms in the alkyl moiety, such as chloromethyl, bromomethyl, fluoromethyl, iodomethyl, 2-chloroethyl, 1-fluoroethyl, 3-chloropropyl, 2-bromopropyl, 1-chloropropyl, 2-chloro-1-methylethyl, 1-bromo-1-methylethyl, 4-iodobutyl, 3-fluorobutyl, 3-chloro-2-methylpropyl, 2-iodo-2-methylpropyl, 1-fluoro-2-methylpropyl, 2-chloro-1,1-dimethylethyl, 2-bromo-1,1-dimethylethyl,

5-bromopentyl, and 4-chlorohexyl.

As the polycyclic aromatic group, there can be exemplified naphthyl, phenanthryl and anthryl.

As the aralkyloxycarbonyl group, there can be exemplified those in which the aralkyl moiety is the above-mentioned variety kinds of aralkyl groups.

As the N-alkylcarbamoyl group, there can be exemplified those in which the alkyl moiety is the above-mentioned variety kinds of alkyl groups.

As the dialkylamino group, there can be exemplified those in which the alkyl moiety is the above-mentioned variety kinds of alkyl groups. The two alkyl groups substituted for the amino groups may be the same or different.

As the substituent that may be substituted for the above-mentioned groups, there can be exemplified a halogen atom, an amino group, a hydroxyl group, a carboxyl group that may be esterified, a cyano group, an alkyl group with 1 tO 6 carbon atoms, an alkoxyl group with 1 to 6 carbon atoms, and an alkenyl group with 2 to 6 carbon atoms that may have an aryl group. There is no particular limitation on the positions of the substituents.

According to the present invention, there can be further used widely known electron-transporting materials, such as benzoquinone compound, malononitrile, thiopyrane compound, tetracyanoethylene, 2,4,8-trinitrothioxanthone, dinitrobenzene, dinitroanthracene, dinitroacridine, nitroanthraquinone, dinitroanthraquinone, anhydrous succinic acid, anhydrous maleic acid or dibromomaleic anhydride, in addition to the above-mentioned materials.

Described below are the charge-generating agent, positive hole-transporting agent and a binder resin used for the electro-photosensitive material of the present invention.

<Charge-Generating Agent>

As the charge-generating agent, there can be exemplified the compounds represented by the following general formulas (CG1) to (CG12).

(CG1) Metal-free phthalocyanine

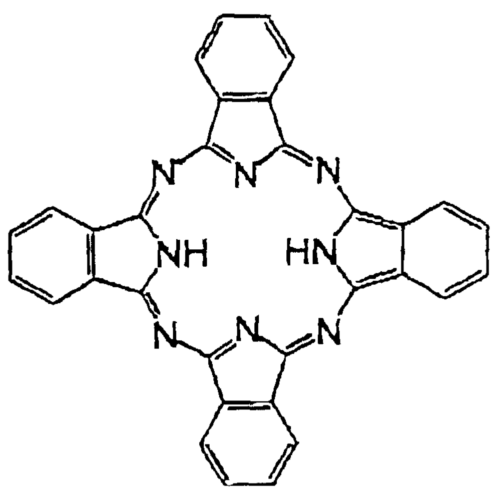

( CG1 )

(CG2) Oxotitanylphthalocyanine

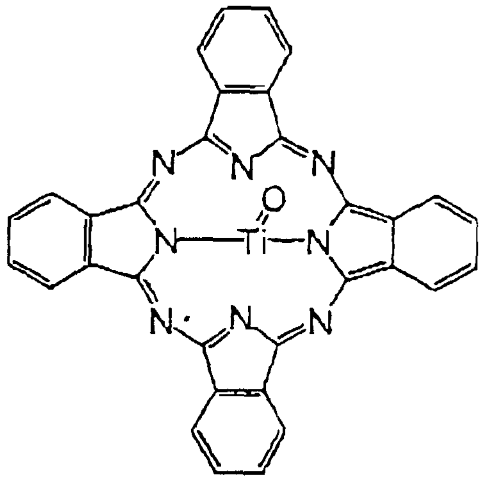

(CG2)

(CG3) Perylene pigment

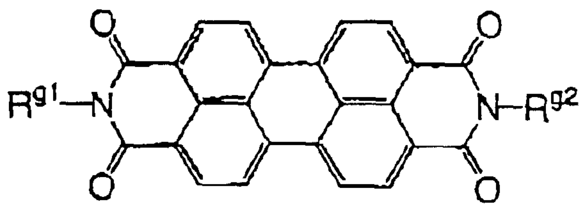

(CG3)

wherein $R^{g1}$ and $R^{g2}$ may be the same or different, and are substituted or unsubstituted alkyl groups, cycloalkyl groups, aryl groups, alkanoyl groups or aralkyl groups with not more than 18 carbon atoms.

(CG4) Bisazo pigment

$$Cp1 - N = N - Q - N = N\ Cp2 \qquad (CG4)$$

wherein Cp1 and Cp2 may be the same or different, and are coupler residues, and Q is a group represented by the formula,

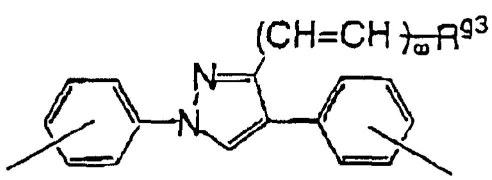

(Q-1)

wherein $R^{g3}$ is a hydrogen atom, an alkyl group, an aryl group or a heterocyclic group, wherein alkyl group, aryl group or heterocyclic group may have a substituent, and ω is 0 or 1,
represented by the formula,

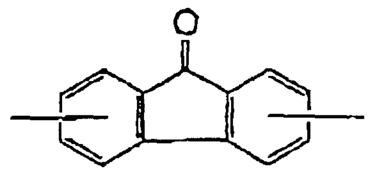

(Q-2)

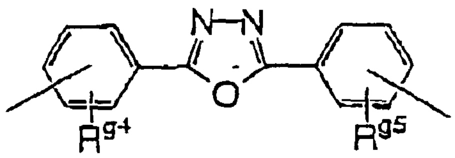

(Q-3)

wherein $R^{g4}$ and $R^{g5}$ may be the same or different, and are hydrogen atoms, alkyl groups with 1 to 5 carbon atoms, halogen atoms, alkoxy group, aryl groups or aralkyl groups,
represented by the formula,

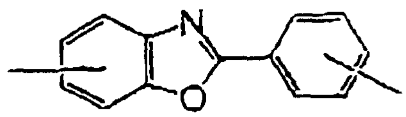

(Q-4)

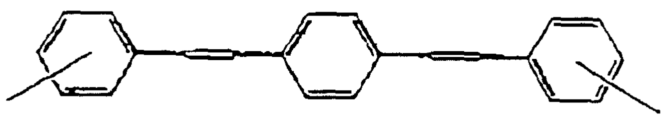

(Q-5)

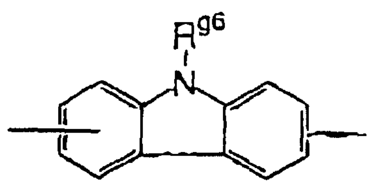

(Q-6)

wherein $R^{g6}$ is a hydrogen atom, an ethyl group, a chloroethyl group or a hydroxyethyl group, or represented by the formula,

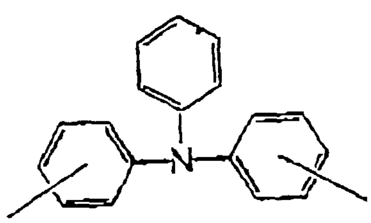

(Q-7)

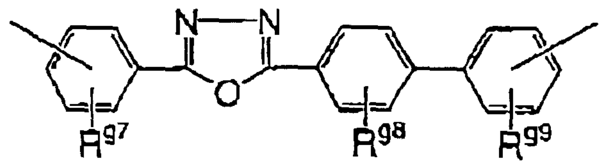

(Q-8)

wherein $R^{g7}$, $R^{g8}$ and $R^{g9}$ which may be the same or different are hydrogen atoms, alkyl groups with 1 to 5 carbon atoms, halogen atoms, alkoxyl groups, aryl groups or aralkyl groups.

(CG5) Dithioketopyrrolopyrrole pigment

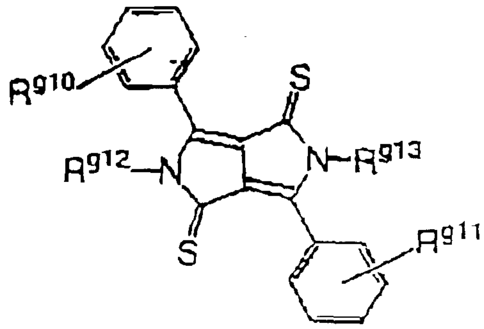

( CG5 )

wherein $R^{g10}$ and $R^{g11}$ may be the same or different and are hydrogen atoms, alkyl groups, alkoxyl groups or halogen atoms, and $R^{g12}$ and $R^{g13}$ may be the same or different and are hydrogen atoms, alkyl groups or aryl groups.

(CG6) Metal-free naphthalocyanine pigment

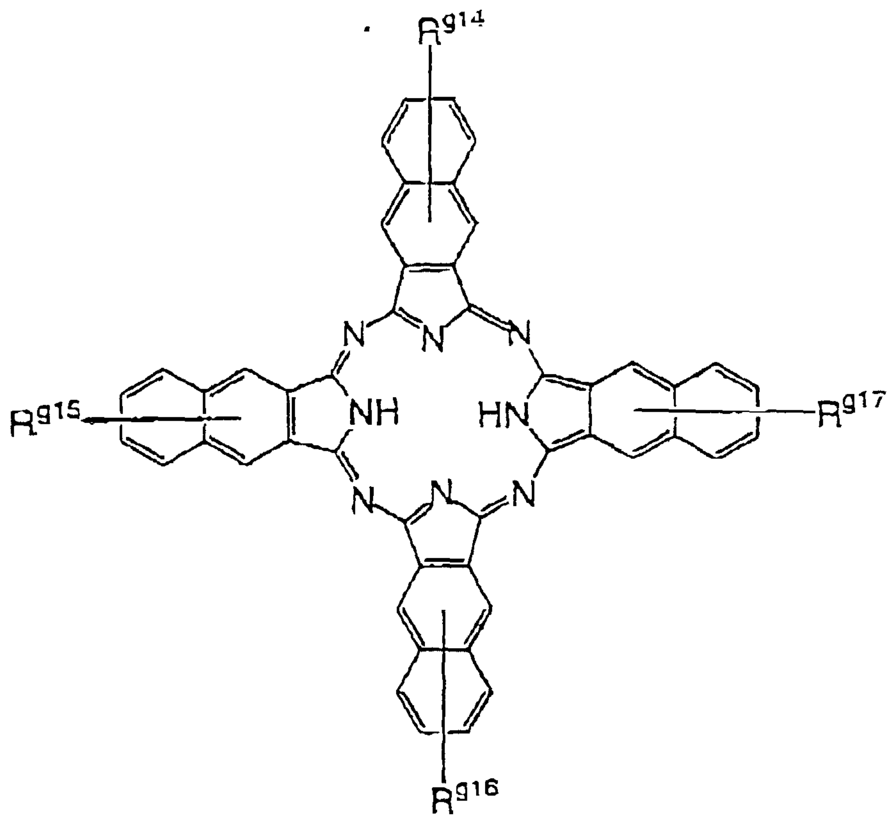

(CG6)

wherein $R^{g14}$, $R^{g15}$, $R^{g16}$ and $Rg^{17}$ may be the same or different, and are hydrogen atoms, alkyl groups, alkoxyl groups or halogen atoms.

(CG7) Metal-free naphthalocyanine pigment

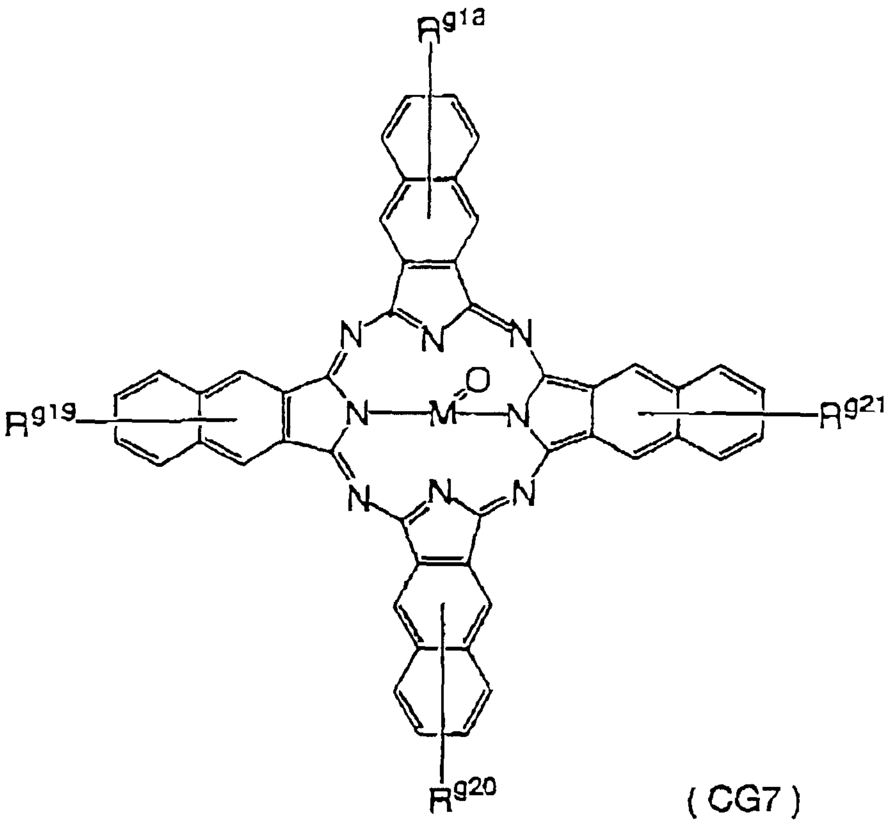

(CG7)

wherein $R^{g18}$, $R^{g19}$, $R^{g20}$ and $R^{g21}$ may be the same or different, and are hydrogen atoms, alkyl groups, alkoxyl groups or halogen atoms, and M is Ti or V.

(CG8) Squalane pigment

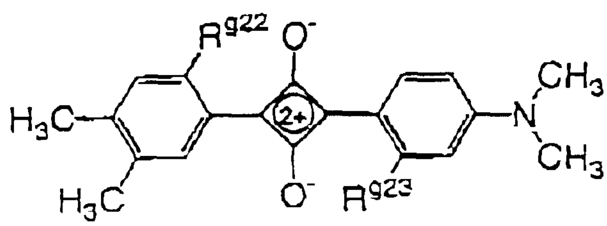

(CG8)

wherein $R^{g22}$ and $R^{g23}$ may be the same or different, and are hydrogen atoms, alkyl groups, alkoxyl groups or halogen atoms.

(CG9) Trisazo pigment

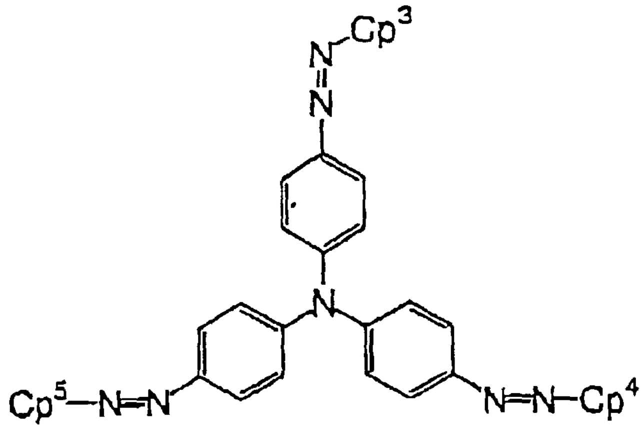

(CG9)

wherein Cp3, Cp4 and Cp5 may be the same or different, and are coupler residues.

(CG10) Indigo pigment

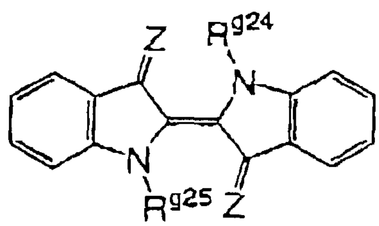

(CG10)

wherein $R^{g24}$ and $R^{g25}$ may be the same or different and are hydrogen atoms, alkyl groups or aryl groups, and Z is an oxygen atom or a sulfur atom.

(CG11) Azulenium pigment

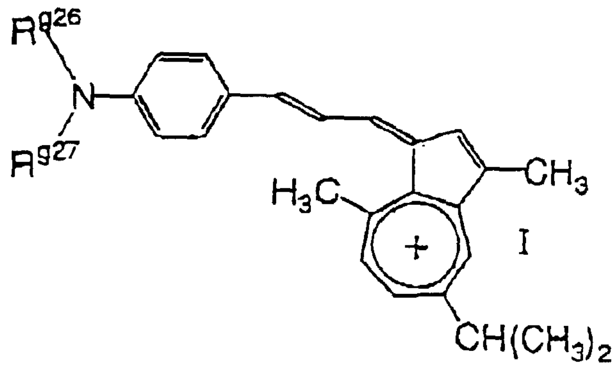

(CG11)

wherein $R^{g26}$ and $R^{g27}$ may be the same or different, and are hydrogen atoms, alkyl groups or aryl groups.

(CG12) Cyanine pigment

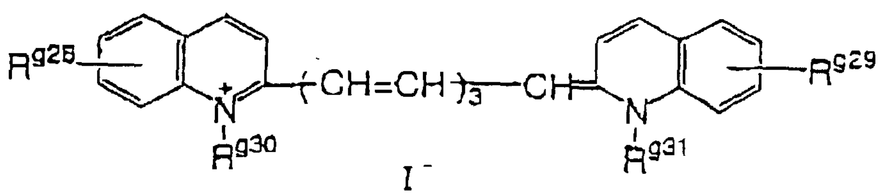

(CG12)

wherein $R^{g28}$ and $R^{g29}$ may be the same or different, and are hydrogen atoms, alkyl groups, alkoxyl groups or halogen atoms, and $R^{g30}$ and $R^{g31}$ may be the same or different, and are hydrogen atoms, alkyl groups or aryl groups.

In the above-mentioned charge-generating agents, examples of the alkyl group include substituted or unsubstituted alkyl groups with not more than 18 carbon atoms, such as octyl, nonyl, decyl, dodecyl, tridecyl, pentadecyl and octadecyl, in addition to the above-mentioned alkyl groups with 1 to 6 carbon atoms.

As the cycloalkyl group, alkoxyl group, alkanoyl group, heterocyclic group, aryl group and aralkyl group, there can be exemplified those same as those described above.

As the substituent that may be substituted for the above-mentioned groups, there can be exemplified a halogen atom, an amino group, a hydroxyl group, a carboxyl group that may be esterified, a cyano group, an alkyl group with 1 to 6 carbon atoms, an alkoxyl group with 1 to 6 carbon atoms, and an alkenyl group with 2 to 6 carbon atoms that may have an aryl group.

As the coupler residues denoted by Cp1, Cp2, Cp3, Cp4 and Cp5, there can be exemplified the groups represented by the following general formulas (Cp-1) to (Cp-11),

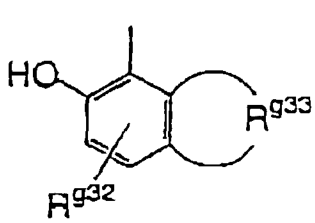

(Cp-1)

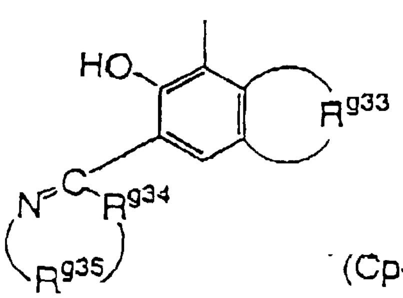

(Cp-2)

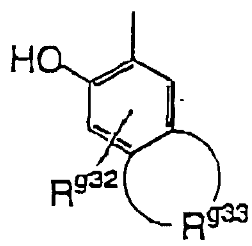

(Cp-3)

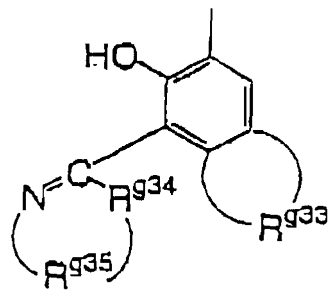

(Cp-4)

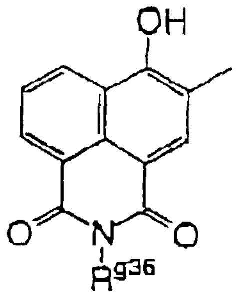

(Cp-5)

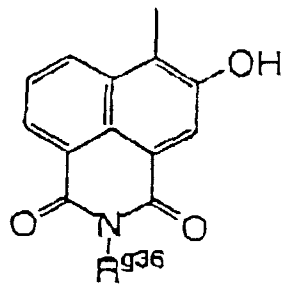

(Cp-6)

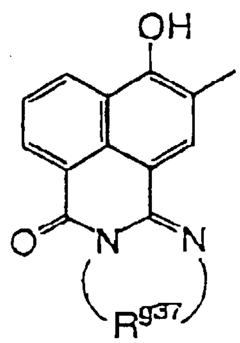

(Cp-7)

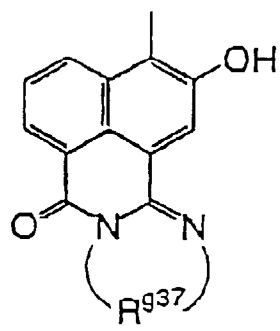

(Cp-8)

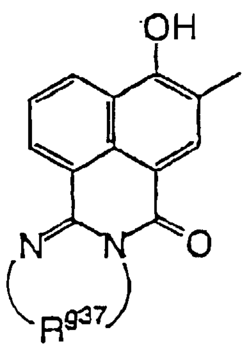

(Cp-9)

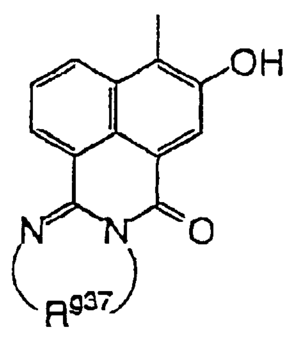

(Cp-10)

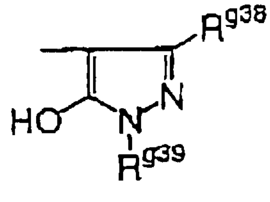

(Cp-11)

wherein $R^{g32}$ is a carbamoyl group, a sulfamoyl group, an allophanoyl group, an oxamoyl group, an anthraniloyl

group, a carbazoyl group, a glycyl group, a hydantoyl group, a phthalamoyl group or a succinamoyl group, each of which may have a substituent such as a halogen atom, a phenyl group that may further have a substituent, a naphthyl group that may further have a substituent, a nitro group, a cyano group, an alkyl group, an alkenyl group, a carbonyl group or a carboxyl group, $R^{g33}$ is an atomic group necessary for forming an aromatic ring, a polycyclic hydrocarbon or a heterocyclic ring being condensed with a benzene ring, and these rings may have a substituent same as those mentioned above, Rg34 is an oxygen atom, a sulfur atom or an imino group, Rg35 is a divalent chain hydrocarbon group or aromatic hydrocarbon group which may have a substituent same as those mentioned above, Rg36 is an alkyl group, an aralkyl group, an aryl group or a heterocyclic ring group which may have a substituent same as those mentioned above, $R^{g37}$ is an atomic group necessary for forming a heterocyclic ring together with the divalent chain hydrocarbon group or aromatic hydrocarbon group, or together with two nitrogen atoms in the groups (Cp-1) to (Cp-11), and these rings may have a substituent same as those mentioned above, $R^{g38}$ is a hydrogen atom, an alkyl group, an amino group, a carbamoyl group, a sulfamoyl group, an allophanoyl group, a carboxyl group, an alkoxycarbonyl group, an aryl group or a cyano group, and the groups other than the hydrogen atom may have a substituent same as those mentioned above, and $R^{g39}$ is an alkyl group or an aryl group which may have a substituent same as those mentioned above.

As the alkenyl group, there can be exemplified those having 2 to 6 carbon atoms such as vinyl, allyl, 2-butenyl, 3-butenyl, 1-methylallyl, 2-pentenyl, and 2-hexenyl.

In the group Rg33, as the atomic group necessary for forming an aromatic ring being condensed with a benzene ring, there can be exemplified those alkylene groups having 1 to 4 carbon atoms, such as methylene, ethylene, trimethylene and tetramethylene.

As the aromatic ring formed by the condensation of the group $R^{g31}$ with a benzene ring, there can be exemplified a naphthalene ring, an anthracene ring, a phenanthrene ring, a pyrene ring, a chrysene ring and a naphthacene ring.

In the group $R^{g33}$, as the atomic group necessary for forming a polycyclic hydrocarbon being condensed with a benzene ring, there can be exemplified the above-mentioned alkylene group having 1 to 4 carbon atoms, as well as a carbazole ring, a benzocarbazole ring and a dibenzofuran ring.

In the group $R^{g33}$, furthermore, as the atomic group necessary for forming a heterocyclic ring being condensed with a benzene ring, there can be exemplified benzofuranyl, benzothiophenyl, indolyl, lH-indolyl, benzoxazolyl, benzothiazolyl, lH-indazolyl, benzimidazolyl, chromenyl, chromanyl, isochromanyl, quinolinyl, isoquinolinyl, cinnolinyl, phthaladinyl, quinozonilyl, quinoxalinyl, dibenzofuranyl, carbazolyl, xanthenyl, acrydinyl, phenanthridinyl, phenadinyl, phenoxadinyl and thianthrenyl.

As the aromatic heterocyclic group formed by the condensation of the group $R^{g33}$ with a benzene ring, there can be exemplified thienyl, furyl, pyrrolyl, oxazolyl, isooxazolyl, thiazolyl, isothiazolyl, imidazolyl, pyrazolyl, triazolyl, tetrazolyl, pyridyl and thiazolyl. It may be a heterocyclic ring (e.g., benzofuranyl, benzimidazolyl, benzoxazolyl, benzothiazolyl or quinolyl) condensed with other aromatic ring.

In the groups $R^{g35}$ and $R^{g37}$, there can be exemplified ethylene, trimethylene or tetramethylene as the divalent chain hydrocarbon group, and there can be exemplified phenylene, naphthylene or phenanthrelene as the divalent aromatic hydrocarbon group.

In the group $R^{g36}$, there can be exemplified pyridyl, pyradyl, thienyl, pyranyl or indolyl as the heterocyclic group.

In the group $R^{g37}$, as the atomic group necessary for forming a heterocyclic ring together with two nitrogen atoms, there can be exemplified phenylene, naphthylene, phenanthrelene, ethylene, trimethylene and tetramethylene.

As the aromatic heterocyclic ring group formed by the group $R^{g37}$ and two nitrogen atoms, there can be exemplified benzimidazole, benzo[f]benzimidazole,
dibenzo[e,g,]benzimidazole and benzopyrimidine. These groups may have a substituent same as those mentioned above.

In the group $R^{g38}$, there can be exemplified methoxycarbonyl, ethoxycarbonyl, propoxycarbonyl and butoxycarbonyl as the alkoxycarbonyl group.

In the present invention, it is allowable to use a powder of inorganic photoconducting material such as selenium, selenium-tellurium, selenium-arsenic, cadmium sulfide, or amorphous silicon, as well as a widely known charge-generating agent such as pyrylium salt, anthanthrone pigment, triphenylmethane pigment, threne pigment, toluidine pigment, pyrazoline pigment or quinacrydone pigment.

The above-mentioned charge-generating agent is used in a single kind or in a combination of two or more kinds so as to absorb waves in a desired region.

The image-forming apparatus of the digital optical system such as a laser beam printer or a facsimile that uses a source of light such as semiconductor laser, requires a photosensitive material that exhibits sensitivity in a wavelength region of noy shorter than 700 nm. Among the above-mentioned charge-generating agents, therefore, there is preferably used a phthalocyanine pigment such as a metal-free phthalocyanine represented by the general formula (CG1) or an oxotitanylphthalocyanine represented by the general formula (CG2). The phthalocyanine pigment of any crystalline shape can be used without any limitation.

On the other hand, the image-forming apparatus of the analog optical type such as an electrostatic copying machine using a source of white light such as halogen lamp, requires a photosensitive material that exhibits sensitivity in the visible region. Therefore, there is preferably used a perylene pigment represented by the general formula (CG3) or a bisazo pigment represented by the general formula (CG4).

<Positive Hole-Transporting Agent>

As the positive hole-transporting agent, there can be used various compounds having a high positive hole-transporting ability, such as the compounds represented by the following formulas (HT1) to (HT13).

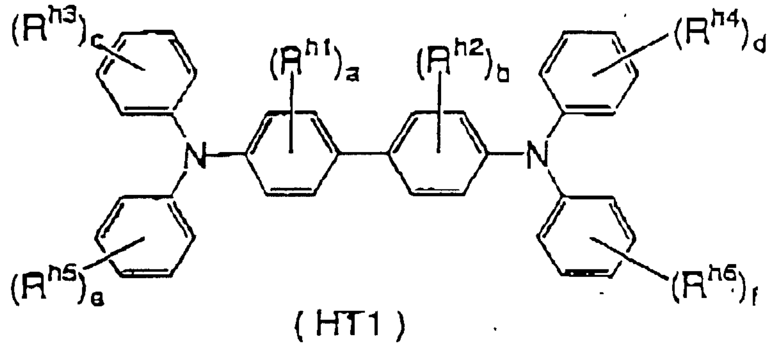

(HT1)

wherein $R^{h1}$, $R^{h2}$, $R^{h3}$, $R^{h4}$, $R^{h5}$ and $R^{h6}$ may be the same or different, and are halogen atoms, alkyl groups that may have a substituent, alkoxyl groups that may have a substituent or aryl groups that may have a substituent, a and b may be the same or different, and are integers of from 0 to 4, and c, d, e and f may be the same or different, and are integers of from 0 to 5, but when a, b, c, d, e or f is 2 or larger, $R^{h1}$, $R^{h2}$, $R^{h3}$, $R^{h4}$, $R^{h5}$ and $R^{h6}$ may not be the same.

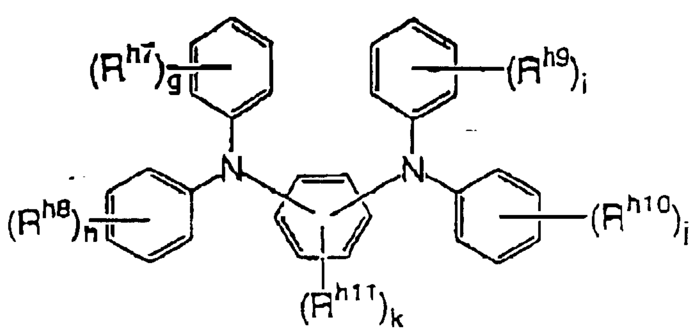

(HT2)

wherein $R^{h7}$, $R^{h8}$, $R^{h9}$, $R^{h10}$ and $R^{h11}$ may be the same or different, and are halogen atoms, alkyl groups that may have a substituent, alkoxyl groups that may have a substituent, or aryl groups that may have a substituent, g, h, i and j may be the same or different, and are integers of from 0 to 5, and k is an integer of from 0 to 4, but when g, h, i, j or k is 2 or larger, $R^{h7}$, $R^{h8}$, $R^{h9}$, $R^{h10}$ and $R^{h11}$ may not be the same.

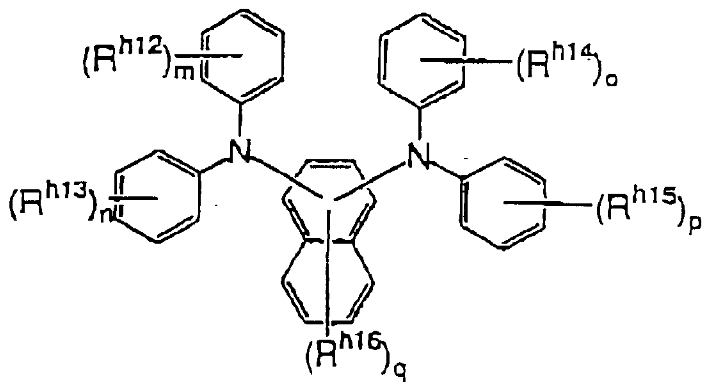

( HT3 )

wherein $R^{h12}$, $R^{h13}$, $R^{h14}$ and $R^{h15}$ may be the same or different, and are halogen atoms, alkyl groups that may have a substituent, alkoxyl groups that may have a substituent or aryl groups that may have a substituent, and $R^{h16}$ is a halogen atom, a cyano group, a nitro group, an alkyl group that may have a substituent, an alkoxyl group that may have a substituent or an aryl group that may have a substituent, m, n, o and p may be the same or different, and are integers of from 0 to 5, and q is an integer of from 1 to 6, but when m, n, o, p or q is 2 or larger, $R^{h12}$, $R^{h13}$, $R^{h14}$, $R^{h15}$ and $R^{h16}$ may not be the same.

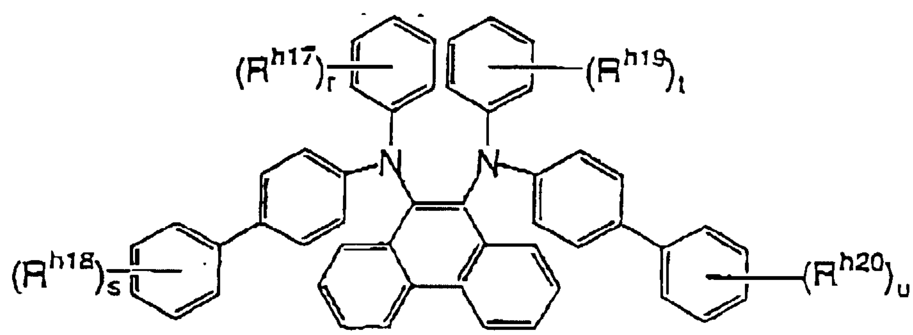

( HT4 )

wherein $R^{h17}$, $R^{h16}$, $R^{h19}$ and $R^{h20}$ may be the same or different, and are halogen atoms, alkyl groups that may have a substituent, alkoxyl groups that may have a substituent or aryl groups that may have a substituent, and r, s, t and u may be the same or different and are integers of 0 to 5, but when r, s, t or u is 2 or larger, $R^{h17}$, $R^{h18}$, $R^{h19}$ and $R^{h20}$ may not be the same,

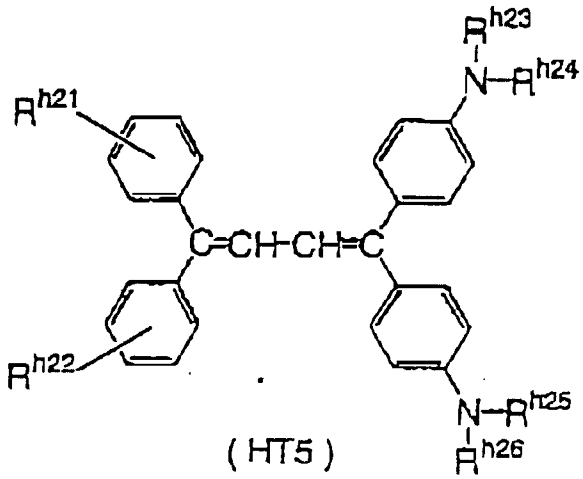

( HT5 )

wherein $R^{h21}$ and $R^{h22}$ may be the same or different, and are hydrogen atoms, halogen atoms, alkyl groups or alkoxyl groups, and $R^{h23}$, $R^{h24}$, $R^{h25}$ and $R^{h26}$ may be the same or different, and are hydrogen atoms, alkyl groups or aryl groups.

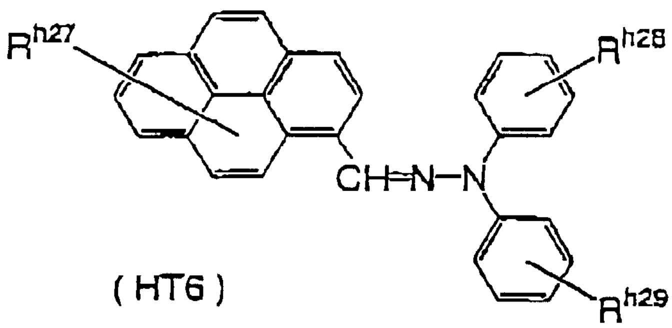

( HT6 )

wherein $R^{h27}$, $R^{h28}$ and $R^{h29}$ may be the same or different, and are hydrogen atoms, halogen atoms, alkyl groups or alkoxyl groups.

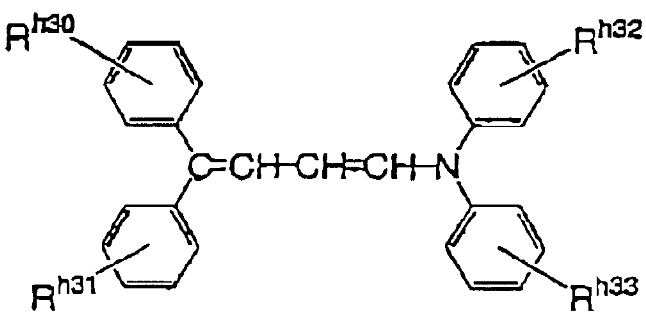

( HT7 )

wherein $R^{h30}$, $R^{h31}$, $R^{h32}$ and $R^{h33}$ may be the same or different and are hydrogen atoms, halogen atoms, alkyl groups or alkoxyl groups.

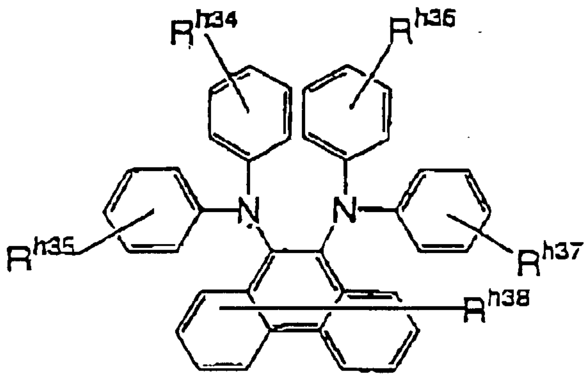

( HT8 )

wherein $R^{h34}$, $R^{h35}$, $R^{h36}$, $R^{h37}$ and $R^{h38}$ may be the same or different and are hydrogen atoms, halogen atoms, alkyl groups or alkoxyl groups.

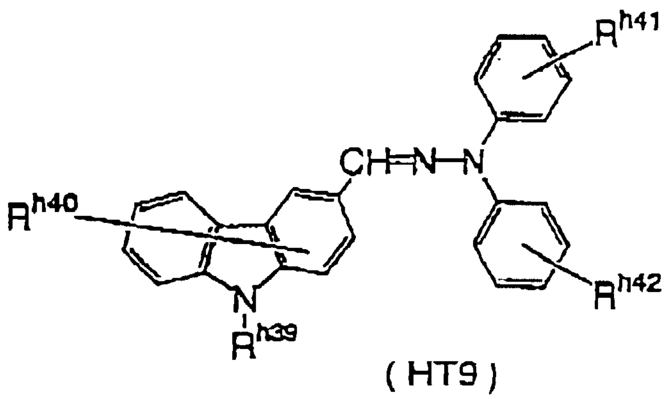

( HT9 )

wherein $R^{h39}$ is a hydrogen atom or an alkyl group, and $R^{h40}$, $R^{h41}$ and $R^{h42}$ may be the same or different, and are hydrogen atoms, halogen atoms, alkyl groups or alkoxyl groups.

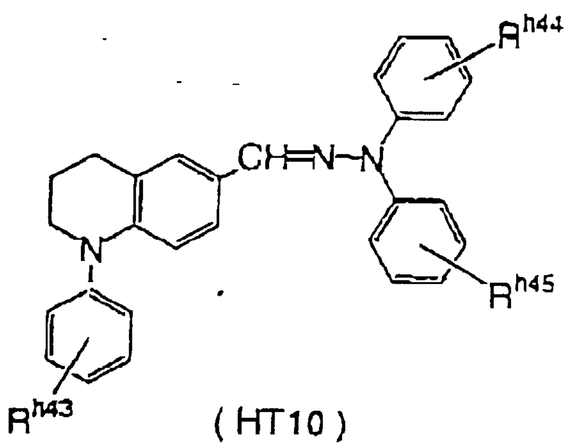

( HT10 )

wherein $R^{h43}$, $R^{h44}$ and $R^{h45}$ may be the same or different and are hydrogen atoms, halogen atoms, alkyl groups or alkoxyl groups.

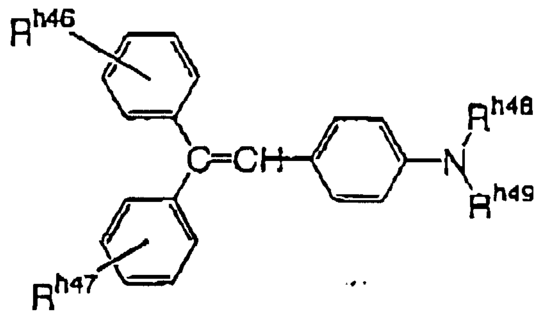

( HT11 )

wherein $R^{h46}$ and $R^{h47}$ may be the same or different, and are hydrogen atoms, halogen atoms, alkyl groups that may have a substituent or alkoxyl groups that may have a substituent, and $R^{h48}$ and $R^{h49}$ may be the same or different and are hydrogen atoms, alkyl groups that may have a substituent or aryl groups that may have a substituent.

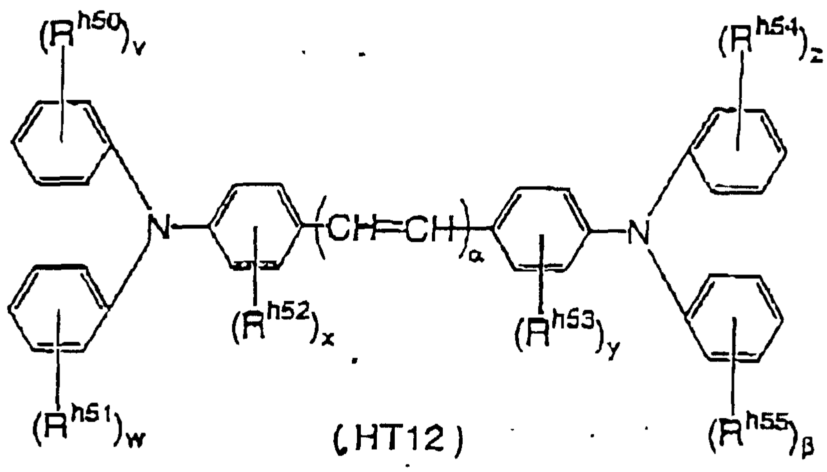

( HT12 )

wherein $R^{h50}$, $R^{h51}$, $R^{h52}$, $R^{h53}$, $R^{h54}$ and $R^{h55}$ may be the same or different, and are alkyl groups that may have a substituent, alkoxyl groups that may have a substituent, or aryl groups that may have a substituent, $\alpha$ is an integer of from 1 to 10, v, w, x, y, z and $\beta$ may be the same or different, and are integers of from 0 to 2, but when v, w, x, y, z or $\beta$ is 2, $R^{h50}$, $R^{h51}$, $R^{h53}$, $R^{h54}$ and $R^{h55}$ may not be the same.

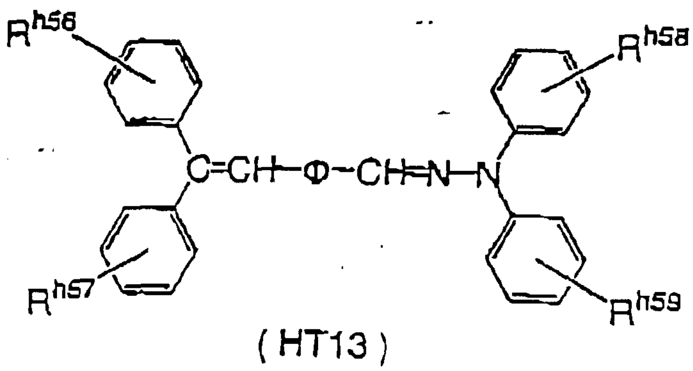

( HT13 )

wherein $R^{h56}$, $R^{h57}$, $R^{h58}$ and $R^{h59}$ may be the same or different, and are hydrogen atoms, halogen atoms, alkyl groups or alkoxyl groups, and $\phi$ is a group ($\phi$-1), ($\phi$-2) or ($\phi$-3) represented by,

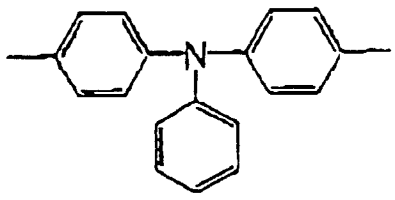

( Φ-1 )

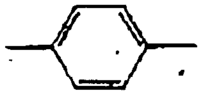

( Φ-2 )

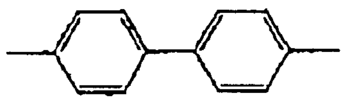

( Φ-3 )

In the above-mentioned positive-hole transporting agent, the alkyl group, alkoxyl group, halogen atom, aryl group and aralkyl group are the same as those described above.

As the substituent which the above-mentioned groups may have, there can be exemplified a halogen atom, an amino group, a hydroxyl group, a carboxyl group that may be esterified, a cyano group, an alkyl group having 1 to 6 carbon atoms, an alkoxyl group having 1 to 6 carbon atoms, and an alkenyl group having 2 to 6 carbon atoms that may have an aryl group. There is no particular limitation on the positions where the substituents are substituted.

In the present invention, there can be further used widely known positive hole-transporting substances, i.e., nitrogen-containing cyclic compounds and condensed polycyclic compounds like oxadiazole compound such as 2,5-di (4-methylaminophenyl)-1,3,4-oxadiazole; styryl compound such as 9-(4-diethylaminostyryl) anthracene; carbazole compound such as polyvinylcarbazole; organopolysilane compound; pyrazoline compound such as 1-phenyl-3-(p-dimethylaminophenyl)pyrazoline; hydrazone compound; triphenylamine compound: indole compound; oxazole compound; isooxazole compound; thiazole compound; thiadiazole compound; imidazole compound; pyrazole compound; and triazole compound.

In the present invention, the positive hole-transporting agent may be used in a single kind or being mixed in two or more kinds. When a positive hole-transporting agent having a film-forming property such as polyvinyl carbazole is used, the binder resin is not necessarily needed.

Among the above-mentioned positive hole-transporting agents according to the present invention, it is desired to use the one having an ionization potential (Ip) of from 4.8 to 5.6 eV, and a mobility of not smaller than 1 x $10^{-6}$ $cm^2$/V·sec in an electric field intensity of 3 x $10^5$ V/cm.

By using the positive-hole transporting agent of which the ionization potential lies within the above-mentioned range, the residual potential further decreases and the sensitivity is improved. Though not yet clear, the reasons are attributed to be as described below.

That is, the easy injection of electric charge from the charge-generating agent into the positive hole-transporting agent is closely related to the ionization potential of the positive hole-transporting agent. When the ionization potential of the positive hole-transporting agent is larger than the above-mentioned range, the degree of injection of electric charge from the charge-generating agent into the positive hole-transporting agent decreases or the degree of exchange of positive holes decreases in the positive hole-transporting agent, resulting in the drop of sensitivity. In a system in which both the positive hole-transporting agent and the electron-transporting agent are present, on the other hand, attention must be given to the interaction between the two or, more concretely, to the formation of a complex for migrating electric charge. When such a complex is formed between the two, recombination occurs between the positive hole and the electron, and the mobility of electric charge drops as a whole. When the ionization potential of the positive hole-transporting agent is smaller than the above-mentioned range, a complex tends to be formed relative to the electron-transporting agent. Therefore, recombination of the electron and the positive hole occurs, resulting in a decrease in the apparent quantum yield and a decrease in the sensitivity.

When a bulky group exists in the electron-transporting agent, the formation of a complex for migrating the charge is suppressed due to the steric hindrance. It is therefore desired to introduce a substituent which is as bulky as possible into the naphthoquinone derivative (1) that is used as an electron-transporting agent in the present invention.

As a concrete example of the positive hole-transporting agent that is preferably used in the present invention, there can be raised a compound represented by the following formula (HT1-1),

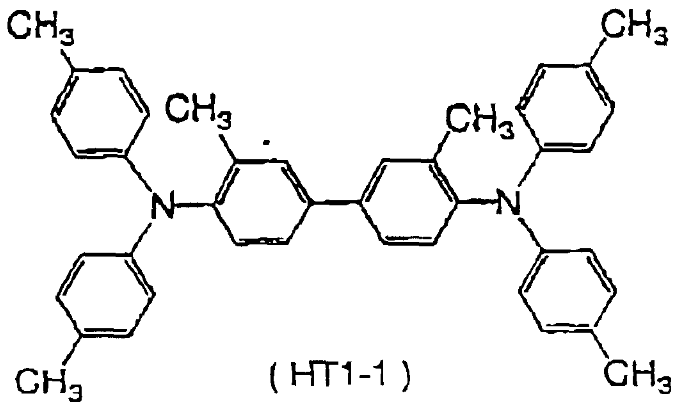

( HT1-1 )

which belongs to a benzidine derivative expressed by the above-mentioned formula (HT1).

<Binder Resin>

As a binder resin for dispersing the above-mentioned components, there can be used a variety of resins that have heretofore been used for the photosensitive layers, i.e., thermoplastic resins such as styrene polymer, styrene-butadiene copolymer, styrene-acrylonitrile copolymer, styrene-maleic acid copolymer, acrylic copolymer, styrene-acrylic acid copolymer, polyethylene, ethylene-vinyl acetate copolymer, chlorinated polyethylene, polyvinyl chloride, polypropylene, ionomer, vinyl chloride-vinyl acetate copolymer, polyester, alkyd resin, polyamide, polyurethane, polycarbonate, polyarylate, polysulfon, diallyl phthalate resin, ketone resin, polyvinyl butylal resin, polyether resin, and polyester resin, as well as silicone resin, epoxy resin, phenol resin, urea resin, melamine resin, and other crosslinked thermosetting resin, and photocurable resins such as epoxy acrylate and urethane-acrylate. These binder resins can be used in a mixture of one or two or more kinds. Preferred examples of the resin include styrene polymer, acrylic polymer, styrene-acrylic copolymer, polyester, alkyd resin, polycarbonate and polyarylate.

Next, described below is a process for producing the electro-photosensitive material of the present invention.

To obtain a single-layer type electro-photosensitive material, a coating solution obtained by dissolving or dispersing a predetermined electron-transporting agent in a suitable solvent together with the charge-generating agent, positive hole-transporting agent and binder resin, is applied onto an electrically conducting substrate by using a coating means and is dried.

In the single-layer type photosensitive material, the charge-generating agent is blended in an amount of from 0.1 to 50 parts by weight and, preferably, from 0.5 co 30 parts by weight, and the electron-transporting agent is blended in an amount of from 5 to 100 parts by weight and, preferably, from 10 to 80 parts by weight per 100 parts by weight of the binder resin. The positive hole-transporting agent is blended in an amount of from 5 to 500 parts by weight and, preferably, from 25 to 200 parts by weight. It is desired that the total amount of the positive hole-transporting agent and the electron-transporting agent is from 20 to 500 parts by weight and, preferably, from 30 to 200 parts by weight per 100 parts by weight of the binder resin. When an electron-accepting compound is contained in the single-layer type photosensitive layer, it is desired that the electron-accepting compound is blended in an amount of from 0.1 to 40 parts by weight and, preferably, from 0.5 to 20 parts by weight per 100 parts by weight of the binder resin.

The single-layer type photosensitive layer has a thickness of from 5 to 100 μm and, preferably, from 10 to 50 μm.

To obtain the laminated-layer type electro-photosensitive material, first, a charge-generating layer containing a charge-generating agent is formed on an electrically conducting substrate by such means as evaporation or coating, and a coating solution containing an electron-transporting agent and a binder resin is applied onto the charge-generating layer by coating means followed by drying to thereby form a charge-transporting layer.

In the laminated-layer type photosensitive material, the charge-generating agent and the binder resin that constitute the charge-generating layer can be used at various ratios, but it is desired that the charge-generating agent is used in an amount of from 5 to 1000 parts by weight and, preferably, from 30 to 500 parts by weight per 100 parts by weight of the binder resin. When an electron-accepting compound is contained in the laminated-layer type photosensitive layer, it is desired that the electron-accepting compound is blended in an amount of from 0.1 to 40 parts by weight and, preferably, from 0.5 to 20 parts by weight per 100 parts by weight of the binder resin. Furthermore, when an electron-transporting agent is contained in the charge-generating layer, it is desired that the electron-transporting agent is blended in an amount of from 0.5 to 50 parts by weight and, preferably, from 1 to 40 parts by weight per 100 parts by weight of the binder resin.

The electron-transporting agent and the binder resin that constitute the charge-transporting layer can be used at various ratios within ranges in which they do not impair the transportation of electric charge and in which they are not crystallized. It is, however, desired that the electron-transporting agent is blended in an amount of from 10 to 500 parts by weight and, preferably, from 25 to 100 parts by weight per 100 parts by weight of the binder resin, so that the electric charge generated in the charge-generating layer upon the irradiation with light can be easily transported. When the electron-accepting compound is contained in the charge-transporting layer, it is desired that the electron-accepting compound is blended in an amount of from 0.1 to 40 parts by weight and, preferably, from 0.5 to 20 parts by weight per 100 parts by weight of the binder resin.

As for the thickness of the laminated-layer type photosensitive layer, the charge-generating layer has a thickness of from about 0.01 to about 5 μm and, preferably, from about 0.1 to about 3 μm, and the charge-transporting layer has a thickness of from about 2 to about 100 μm and, preferably, from about 5 to about 50 μm.

The single-layer type photosensitive material may have a barrier layer formed between the electrically conducting substrate and the photosensitive layer, and the laminated-layer type photosensitive material may have a barrier layer formed between the electrically conducting substrate and the charge-generating layer, between the electrically conducting substrate and the charge-transporting layer, or between the charge-generating layer and the charge-transporting layer, the barrier layer having a thickness within a range in which it does not impair the properties of the photosensitive material. Moreover, a protection layer may be formed on the surface of the photosensitive material.

The single-layer type and the laminated-layer type photosensitive layers may be blended with a variety of widely known additives such as antioxidant, radical-trapping agent, singlet quencher, degradation-preventing agent like ultraviolet ray-absorbing agent, as well as softening agent, plasticizer, surface-reforming agent, filler, viscosity-increasing agent, dispersion stabilizer, wax, acceptor and donor. In order to improve the sensitivity of the photosensitive layer, furthermore, there may be used widely known sensitizers such as terphenyl, halonaphthoquinones and acenaphthylene together with the charge-generating agent.

Moreover, the photosensitive layer may contain a variety of electron-transporting agents having a high electron-transporting ability together with the naphthoquinone derivative represented by the above-mentioned general formula (1).

As the electrically conducting substrate used for the photosensitive material of the present invention, there can be used a variety of materials having electrically conducting property, such as aluminum, iron, copper, tin, platinum, silver, vanadium, molybdenum, chromium, cadmium, titanium, nickel, palladium, indium, stainless steel, and brass, which are simple metals, as well as a plastic material on which the above-mentioned metals are vaporized or laminated, and a glass coated with aluminum iodide, tin oxide or indium oxide.

The electrically conducting substrate may be in the form of either a sheet or a drum. What is important is that the substrate itself has electrically conducting property, or the surface of the substrate has electrically conducting property. It is desired that the electrically conducting substrate has a sufficiently large mechanical strength.

The photosensitive layer of the present invention is produced by coating the electrically conducting substrate with a coating solution obtained by dissolving or dispersing a resin composition containing the above-mentioned components in a solvent, followed by drying.

That is, the above-mentioned charge-generating agent, charge-transporting agent and a binder resin are dispersed and mixed in a suitable solvent relying on a widely known method such as roll mill, ball mill, Atritor, paint shaker or ultrasonic wave dispersing machine to prepare a coating solution which is then applied by a known means and is dried.

A variety of organic solvents can be used for preparing a coating solution, i.e., alcohols such as methanol, ethanol, isopropanol and butanol; aliphatic hydrocarbons such as n-hexane, octane and cyclohexane; aromatic hydrocarbons such as benzene, toluene and xylene; halogenated hydrocarbons such as dichloromethane, dichloroethane, carbon tetrachloride and chlorobenzene; ethers such as dimethyl ether, diethyl ether, tetrahydrofuran, ethylene glycol dimethyl ether and diethylene glycol dimethyl ether; ketones such as acetone, methyl ethyl ketone and cyclohexanone; esters such as ethyl acetate and methyl acetate; as well as dimethylformaldehyde, dimethylformamide and dimethylsulfoxide. These solvents may be used in one kind or being mixed in two or more kinds together.

It is further allowable to use a surfactant and a leveling agent in order to improve dispersion of the charge-transporting material and the charge-generating material, and to improve smoothness on the surface of the photosensitive layer.

## EXAMPLES

The electro-photosensitive material of the present invention will now be described by way of Synthesizing Examples and Examples.

Synthesizing Example 1

Synthesis of a bis(phenylnaphthoquinone) derivative represented by the above-mentioned formula (1-1).

A 2-phenyl-1,3-indandione (4-1) (0.06 mols, 13 g) of the above-mentioned formula (6) in which $R^1$ is a phenyl group and 1.2 equivalent of sodium hydride (60%) were added into a four-way flask to which a refluxing tube is connected. The interior of the flask was deaerated to a sufficient degree, dried, and was substituted with an argon gas. Then, the flask was cooled with ice, and into which 200 ml of an anhydrous tetrahydrofuran was slowly and dropwisely added.

The mixture was stirred until hydrogen no longer evolved from the solution, and a tetrahydrofuran solution of a diacetylbenzene derivative (0.5 equivalent) represented by the above-mentioned formula (4-2) was dropwisely added thereto. The solution was stirred for 4 hours while being refluxed, and was reacted while being monitored until the spots of bromo-compound have disappeared. After the reaction, the hydrochloric acid water was added to the solution, and the formed precipitate was separated by filtration and was extracted with chloroform. The thus separated chloroform layer was dried on anhydrous sodium sulfate. Thereafter, the solvent was distilled off under a reduced pressure, and the obtained residue was precipitated in a mixture solution of tetrahydrofuran-hexane to thereby obtain a 3-phenyl-1,4-indandione derivative represented by the above-mentioned formula (4-3).

Next, a 3-phenyl-1,4-indandione derivative represented by the above-mentioned formula (4-3) and 1.2 equivalent of sodium hydride (60%) were added into an eggplant type flask to which a refluxing tube is connected. The interior of the flask was deaerated to a sufficient degree, dried, and was substituted with an argon gas. Then, the flask was cooled with ice, and into which 200 ml of the anhydrous tetrahydrofuran was added. The tetrahydrofuran solution was stirred for 5 hours with refluxing and was reacted while being monitored until the spots of the 3-phenyl-1,4-indandione derivative represented by the above-mentioned formula (4-3) have disappeared. After the reaction, the hydrochloric acid water was added to the solution, and the formed precipitate was separated by filtration and was extracted with chloroform. Thereafter, the thus separated chloroform layer was dried on anhydrous sodium sulfate, and the solvent was distilled off under a reduced pressure to obtain a coarse product. Next, this coarse product, 1 equivalent of silver oxide and 300 ml of chloroform were added into the 500-ml Erlenmeyer flask, and the mixture was stirred at room temperature for 5 hours, the solid product precipitated in the solution was removed, the solution was condensed, and the residue was purified through a column chromatography. The solid product was further recrystallized to obtain the captioned product (three-step yield, 4%; melting point, 300°C or higher).

Fig. 2 shows a 1H-NMR spectrum of the product, Fig. 3 shows an IR spectrum, and Fig. 4 shows an MS spectrum.

The above-mentioned reactions are represented by the following reaction formulas,

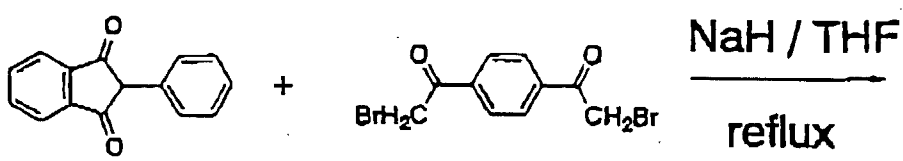


(4-1) (4-2)

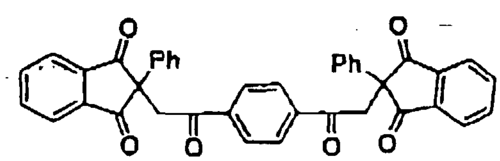

(4-3)

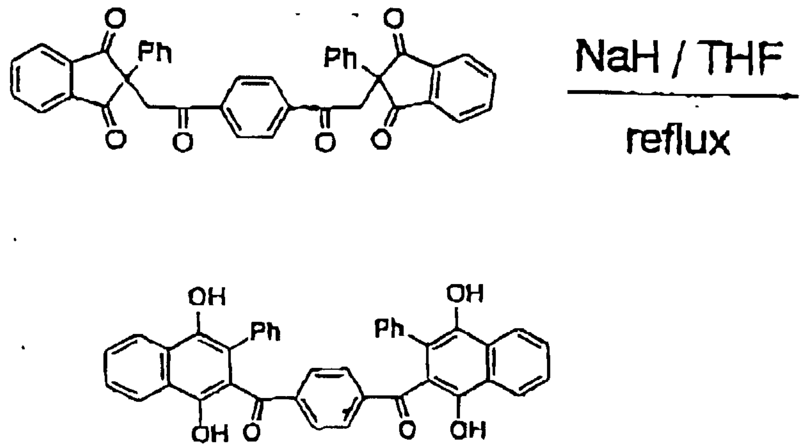


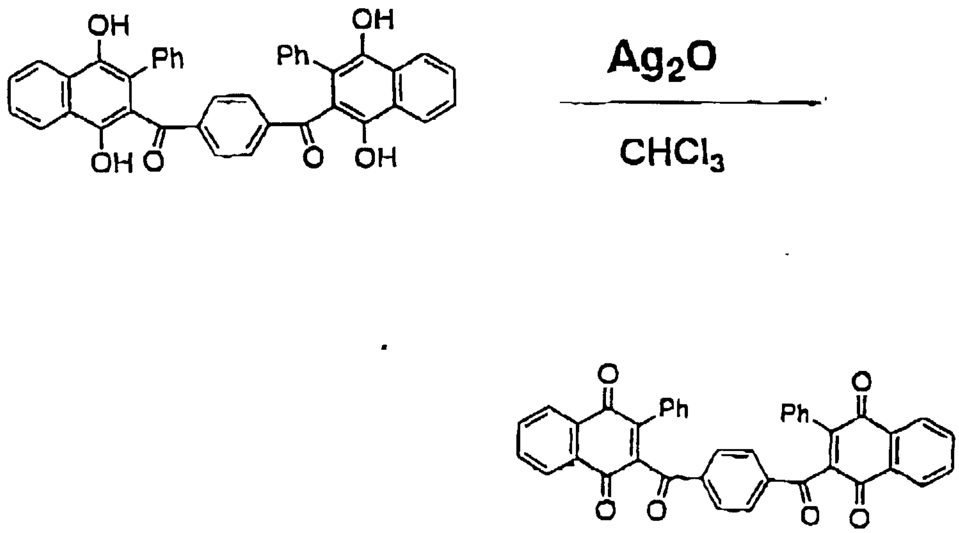


Synthesis Example 2

Synthesis of a bis(phenylnaphthoquinone) derivative represented by the above-mentioned formula (1-2).

The reaction was carried out in the same manner as in Synthesis Example 1 but using an equal molar amount of a 2-(P-isopropylphenyl)-1,3-indandione (5-1) represented by the above-mentioned formula (6) in which the phenyl group is a 4-isopropylphenyl group, to obtain the captioned compound (three-step yield, 14%; melting point, 276 - 278°C).

Fig. 5 shows a 1H-NMR spectrum of the product, and Fig. 6 shows an IR spectrum.

The compound (5-1) is represented by the following formula.

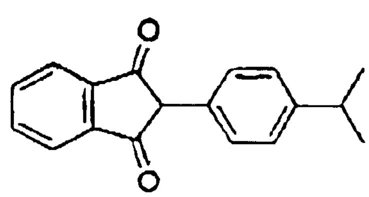

**(5-1)**

Synthesis Example 3

Synthesis of a bis(phenylindandione) derivative represented by the above-mentioned formula (1-3).

The reaction was carried out in the same manner as in Synthesis Example 1 but using an equal molar amount of a 3-(m-methylphenyl)-1,4-indandione (6-1) of the above-mentioned formula (6) in which the phenyl group is a 3-methylphenyl group, to obtain the captioned compound (three-step yield, 9%; melting point, 293 - 295°C).

Fig. 7 shows a 1R-NMR spectrum of the product, and Fig. 8 shows an IR spectrum.

The compound (6-1) is represented by the following formula,

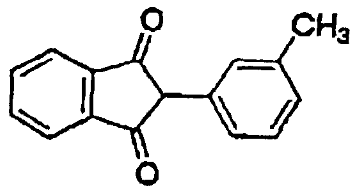

(6-1)

Synthesis Example 4

Synthesis of a bis(phenylindandione) derivative represented by the above-mentioned formula (1-4).

The reaction was carried out in the same manner as in Synthesis Example 1 but using an equal molar amount of a diacetylbiphenyl derivative represented by the formula (4-4) instead of using the diacetylbenzene derivative represented by the above-mentioned formula (4-2), to obtain the captioned compound (three-step yield, 21%; melting point, 300°C or higher).

Fig. 9 shows a 1H-NMR spectrum of the product, Fig. 10 shows an IR spectrum, and Fig. 11 shows an MS spectrum.

The compound (4-4) is represented by the following formula (4-4).

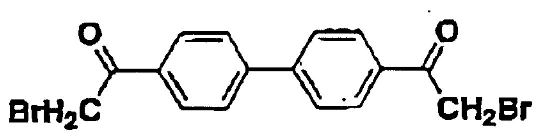

(4-4)

[Preparation of the Electro-Photosensitive Material]

Described below are the components used for the electro-photosensitive material of the present invention.

(i) Charge-generating agent.

$PcH_2$: X-type metal-free phthalocyanine represented by the above-mentioned formula (CG1) [ionization potential (Ip) = 5.38 eV]

PcTiO: oxotitanylphthalocyanine represented by the above-mentioned formula (CG2) (Ip = 5.32 eV)

Perylene: perylene pigment (Ip = 5.50 eV) represented by the following formula (CG3-1)

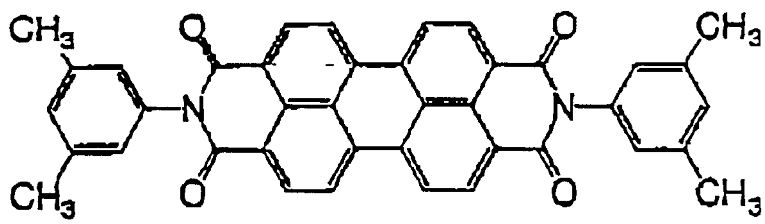

( CG3-1 )

which belongs to the above-mentioned general formula (CG3).

(ii) Positive hole-transporting agent.

HT1-1: Benzidine derivative represented by the above-mentioned formula (HT1-1)(Ip = 5.56 eV)

(iii) Electron-transporting agent.

1-1: naphthoquinone derivative represented by the above-mentioned formula (1-1)
1-2: naphthoquinone derivative represented by the above-mentioned formula (1-2)
1-3: naphthoquinone derivative represented by the above-mentioned formula (1-3)
1-4: naphthoquinone derivative represented by the above-mentioned formula (1-4)

ET13-1: 3-phenyl-1,4-naphthoquinone represented by the following formula (ET13-1),

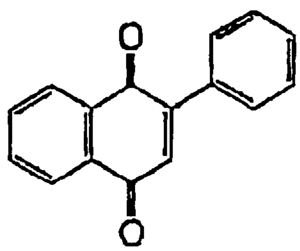

which is disclosed in Japanese Laid-Open Patent Publication No. 110227/1994 and which pertains to the naphthoquinone derivative represented by the above-mentioned formula (ET13).

3-1: p-benzoquinone represented by the above-mentioned formula (3-1)(oxidation-reduction potential = -0.81 V)
3-2: 2,6-di-t-butyl-p-benzoquinone represented by the above-mentioned formula (3-2)(oxidation-reduction potential = -1.30 V)
2-1: 3,5-dimethyl-3',5'-di-t-butyl-4,4'-diphenoquinone represented by the above-mentioned formula (2-1)(oxidation-reduction potential = -0.86 V)
2-2: 3,3',5,5'-tetra-t-butyl-4,4'-diphenoquinone represented by the above-mentioned formula (2-2)(oxidation-reduction potential = -0.94 V)

The ionization potential was measured in the open atmosphere by using a photoelectronic analyzer (AC-1. manufactured by Riken Keiki Co.).

[Preparation of the Single-Layer Type Electro-Photosensitive Material]

Examples 1 to 12 and Comparative Examples 1 to 8

The charge-generating agents, positive hole-transporting agents and electron-transporting agents shown in Tables 1 and 3 were blended together with a binder resin and a solvent at a ratio shown below, and were mixed and dispersed by using a ball mill for 50 hours to prepare coating solutions for the single-layer type photosensitive layers.

| Components | Parts by weight |
|---|---|
| Charge-generating agent | 5 |
| Positive hole-transporting agent | 50 |
| Electron-transporting agent | 30 |
| Binder resin (polycarbonate) | 100 |
| Solvent (tetrahydrofuran) | 800 |

The above-coating solution was applied by a dip-coating method onto the surface of an aluminum blank pipe which is an electrically conducting substrate, and was dried with the hot air heated at 100°C for 60 minutes to prepare a single-layer type electro-photosensitive material having a thickness of 15 to 20 μm.

Examples 21 to 36 and Comparative Examples 13 to 16

Single-layer type electro-photosensitive materials were prepared in the same manner as in the above-mentioned Examples 1 to 3 with the exception of preparing the single-layer type photosensitive layer coating solutions by adding 5 parts by weight of the charge-generating agents, 50 parts by weight of the positive hole-transporting agents, and 30 parts by weight of the electron-transporting agents shown in Tables 2 and 3, and 100 parts by weight of the binder resin and 800 parts by weight of the solvent, and further blending 10 parts by weight of the electron-transporting agents having predetermined reduction potentials represented by the above-mentioned formulas (2-1) to (2-2) and (3-1) to (3-2).

[Preparation of the Laminated-Layer type Electro-Photosensitive Materials]

Examples 13 to 20 and Comparative Examples 9 to 12

100 Parts by weight of the charge-generating agents shown in Tables 1 and 3, 100 parts by weight of the binder resin (polyvinyl butyral) and 2000 parts by weight of the solvent (tetrahydrofuran) were mixed and dispersed by using a ball mill for 50 hours to prepare coating solutions for forming charge-generating layers. The coating solution was applied by the dip-coating method onto the surface of an aluminum blank pipe which is an electrically conducting substrate, and was dried with the hot air heated at 100°C for 60 minutes to form charge-generating layer having a thickness of 1 μm.

Then, 100 parts by weight of the electron-transporting agents shown in Tables 1 to 3, 100 parts by weight of the binder resin (polycarbonate) and 800 parts by weight of the solvent (toluene) were mixed and dispersed using the ball mill for 50 hours to prepare coating solutions for forming charge-transporting layers. The coating solution was applied by the dip-coating method onto the charge-generating layer, and was dried with the hot air heated at 100°C for 60 minutes to form a charge-transporting layer having a thickness of 20 μm to thereby prepare a laminated-layer type electro-photosensitive material.

(Evaluation of Properties of Photosensitive Materials)

The electro-photosensitive materials obtained in the above-mentioned Examples and Comparative Examples were tested for their photo-sensitivities and were evaluated for their sensitivities.

Testing the Photo-Sensitivity.

The photosensitive materials of the above-mentioned Examples and Comparative Examples were applied with a voltage and were electrically charged into +700 v by using a drum-type sensitivity testing machine manufactured by GENTEC Co. Then, the photosensitive materials were exposed to light, and the potentials on the surfaces of the photosensitive materials 330 milliseconds after the exposure to light were measured as post-exposure potentials VL (V).

The irradiation conditions differed depending upon the charge-generating agent of the phthalocyanine type and the charge-generating agent of the perylene type as described below.

(1) Phthalocyanine type pigment.

The surface of the photosensitive material electrically charged to +700 V was irradiated, for 80 milliseconds, with light (halogen lamp, intensity of light: 16 $\mu W/cm^2$) that had been transformed into a monochromatic light of

780 nm (half-value width of 20 nm) through a band-pass filter.

(2) Perylene pigment.

The surface of the photosensitive material electrically charged to +700 V was irradiated with white light (light intensity: 147 μW/cm$^2$) of a halogen lamp for 50 milliseconds.

Tables 1 to 3 show the components used in the above-mentioned Examples and Comparative Examples and the measured results of post-exposure potentials VL.

Table 1

| | Charge generating agent agent | Hole transporting | Electron transporting agent | | VL (V) |
|---|---|---|---|---|---|
| Example 1 | $PcH_2$ | HT1-1 | 1-1 | - | 209 |
| Example 2 | PcTiO | HT1-1 | 1-1 | - | 226 |
| Example 3 | Perylene | HT1-1 | 1-1 | - | 245 |
| Example 4 | $PcH_2$ | HT1-1 | 1-2 | - | 206 |
| Example 5 | PcTiO | HT1-1 | 1-2 | - | 222 |
| Example 6 | Perylene | HT1-1 | 1-2 | - | 240 |
| Example 7 | $PcH_2$ | HT1-1 | 1-3 | - | 207 |
| Example 8 | PcTiO | HT1-1 | 1-3 | - | 224 |
| Example 9 | Perylene | HT1-1 | 1-3 | - | 243 |
| Example 10 | $PcH_2$ | HT1-1 | 1-4 | - | 207 |
| Example 11 | PcTiO | HT1-1 | 1-4 | - | 220 |
| Example 12 | Perylene | HT1-1 | 1-4 | - | 243 |
| Example 13 | $PcH_2$ | - | 1-1 | - | 320 |
| Example 14 | Perylene | - | 1-1 | - | 333 |
| Example 15 | $PcH_2$ | - | 1-2 | - | 313 |
| Example 16 | Perylene | - | 1-2 | - | 325 |
| Example 17 | $PcH_2$ | - | 1-3 | - | 315 |
| Example 18 | Perylene | - | 1-3 | - | 327 |
| Example 19 | $PcH_2$ | - | 1-4 | - | 310 |
| Example 20 | Perylene | - | 1-4 | - | 330 |

Table 2

| | Charge generating agent | Hole transporting agent | Electron transporting agent | | VL (V) |
|---|---|---|---|---|---|
| Example 21 | $PCH_2$ | HT1-1 | 1-1 | 3-1 | 172 |
| Example 22 | $PCH_2$ | HT1-1 | 1-1 | 3-2 | 170 |
| Example 23 | $PCH_2$ | HT1-1 | 1-1 | 2-1 | 168 |
| Example 24 | $PCH_2$ | HT1-1 | 1-1 | 2-2 | 163 |
| Example 25 | $PCH_2$ | HT1-1 | 1-2 | 3-1 | 168 |
| Example 26 | $PCH_2$ | HT1-1 | 1-2 | 3-2 | 165 |
| Example 27 | $PCH_2$ | HT1-1 | 1-2 | 2-1 | 163 |
| Example 28 | $PCH_2$ | HT1-1 | 1-2 | 2-2 | 160 |
| Example 29 | $PCH_2$ | HT1-1 | 1-3 | 3-1 | 169 |
| Example 30 | $PcH_2$ | HT1-1 | 1-3 | 3-2 | 168 |
| Example 31 | $PCH_2$ | HT1-1 | 1-3 | 2-1 | 165 |
| Example 32 | $PCH_2$ | HT1-1 | 1-3 | 2-2 | 162 |
| Example 33 | $PCH_2$ | HT1-1 | 1-4 | 3-1 | 171 |
| Example 34 | $PCH_2$ | HT1-1 | 1-4 | 3-2 | 163 |
| Example 35 | $PCH_2$ | HT1-1 | 1-4 | 2-1 | 165 |
| Example 36 | $PCH_2$ | HT1-1 | 1-4 | 2-2 | 161 |

Table 3

| | Charge generating agent | Hole transporting agent | Electron transporting agent | | VL (V) |
|---|---|---|---|---|---|
| Comp.Ex.1 | $PcH_2$ | HT1-1 | ET13-1 | - | 305 |
| Comp.Ex.2 | PcTiO | HT1-1 | ET13-1 | - | 330 |
| Comp.Ex.3 | Perylene | HT1-1 | ET13-1 | - | 375 |
| Comp.Ex.4 | $PcH_2$ | HT1-1 | 2-1 | - | 220 |
| Comp.Ex.5 | PcTiO | HT1-1 | 2-1 | - | 242 |
| Comp.Ex.6 | $PcH_2$ | HT1-1 | - | - | 478 |
| Comp.Ex.7 | Perylene | HT1-1 | 2-1 | - | 294 |
| Comp.Ex.8 | Perylene | HT1-1 | - | - | 521 |
| Comp.Ex.9 | $PCH_2$ | - | ET13-1 | - | 409 |
| Comp.Ex.10 | perylene | - | ET13-1 | - | 455 |
| Comp.Ex.11 | $PcH_2$ | - | 2-1 | - | 346 |
| Comp.Ex.12 | perylene | - | 2-1 | - | 386 |
| Comp.Ex.13 | $PcH_2$ | HT1-1 | ET13-1 | 3-1 | 295 |
| Comp.Ex.14 | $PcH_2$ | HT1-1 | ET13-1 | 3-2 | 290 |
| Comp.Ex.15 | $PcH_2$ | HT1-1 | ET13-1 | 2-1 | 290 |
| Comp.Ex.16 | $PcH_2$ | HT1-1 | ET13-1 | 2-2 | 288 |

As will be obvious from Tables 1 to 3, the photosensitive materials of Examples exhibit lower post-exposure potentials and higher sensitivities than those of the photosensitive materials of Comparative Examples that use conventional electron-transporting agents or use no electron-transporting agent.

Moreover, the photosensitive materials of Examples 21 to 36, containing an electron-transporting agent having a predetermined oxidation-reduction potential and a naphthoquinone derivative (1) of the present invention, exhibit further decreased post-exposure potentials and further increased sensitivities compared with those of the photosensitive materials of other Examples.

The naphthoquinone derivative (1) used for the electro-photosensitive materials of the present invention exhibits a high electron-transporting ability. Therefore, the electro-photosensitive materials of the present invention containing the naphthoquinone derivative (1) as an electron-transporting agent exhibit markedly decreased residual potentials and high sensitivities.

Upon adding an electron-transporting agent having a particular oxidation-reduction potential, furthermore, there is obtained a photosensitive material exhibiting a further decreased residual potential and increased sensitivity.

Use of the photosensitive material of the present invention makes it possible to operate the copiers and printers at higher speeds.

## Claims

1. A naphthoquinone derivative represented by the general formula (1),

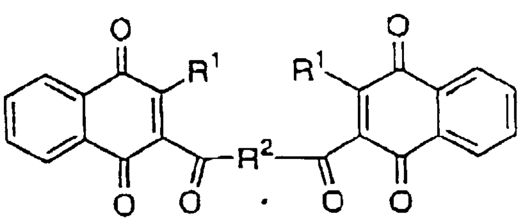

(1)

wherein $R^1$ is an aryl group which may have a substituent, and $R^2$ is an alkylene group which may have a substituent, an arylene group which may have a substituent, a group (i),

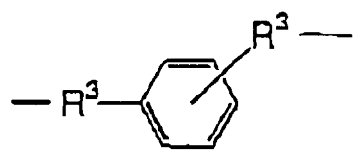

( i )

wherein $R^3$ is an alkylene group which may have a substituent,
or a group (ii),

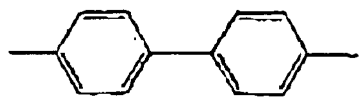

( ii )

2. An electro-photosensitive material comprising a photosensitive layer containing a naphthoquinone derivative of claim 1 formed on an electrically conducting substrate.

3. An electro-photosensitive material according to claim 2, wherein said photosensitive layer contains an electron-transporting agent having an oxidation-reduction potential of from -0.8 to -1.4 V.

4. An electro-photosensitive material according to claim 3, wherein said electron-transporting agent is a diphenoquinone derivative represented by the general formula (2),

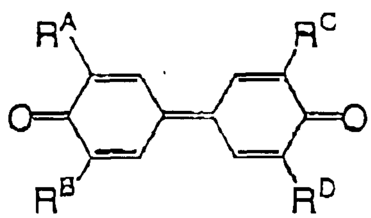

(2)

wherein $R^A$, $R^B$, $R^C$ and $R^D$ may be the same or different, and are hydrogen atoms, alkyl groups, alkoxyl groups, aryl groups, aralkyl groups, cycloalkyl groups or amino groups,
or is a benzoquinone derivative represented by the general formula (3),

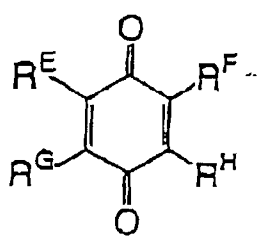

(3)

wherein $R^E$, $R^F$, $R^G$ and $R^H$ may be the same or different, and are hydrogen atoms, alkyl groups, alkoxyl groups, aryl groups, aralkyl groups, cycloalkyl groups or amino groups that may have a substituent.

FIG.1

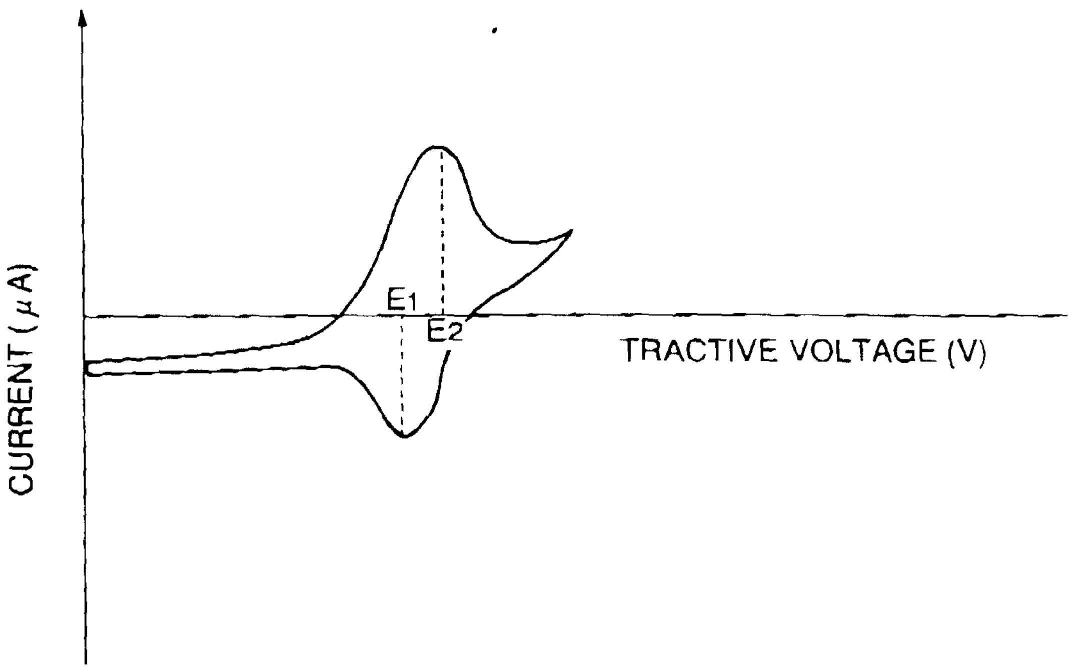

FIG. 2

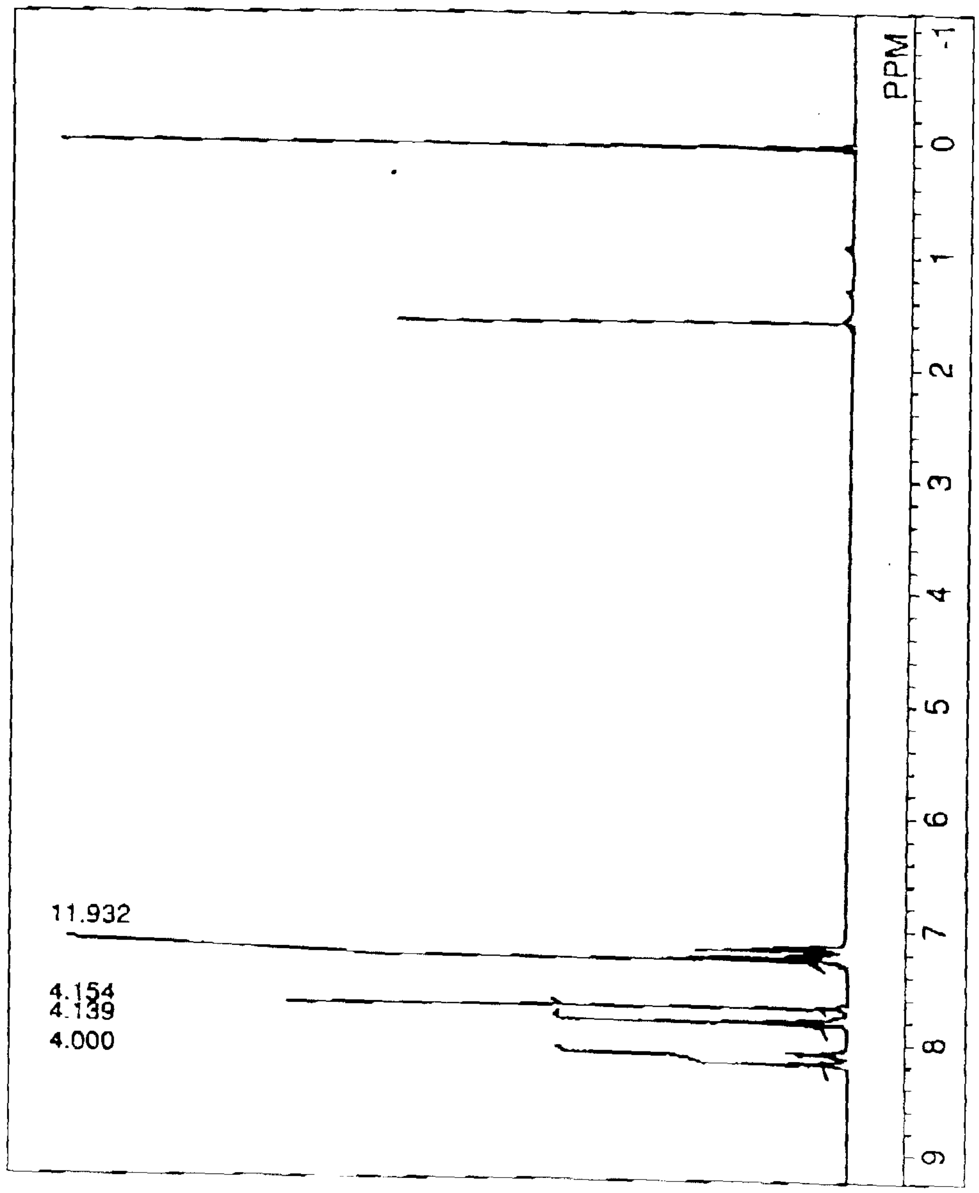

FIG.3

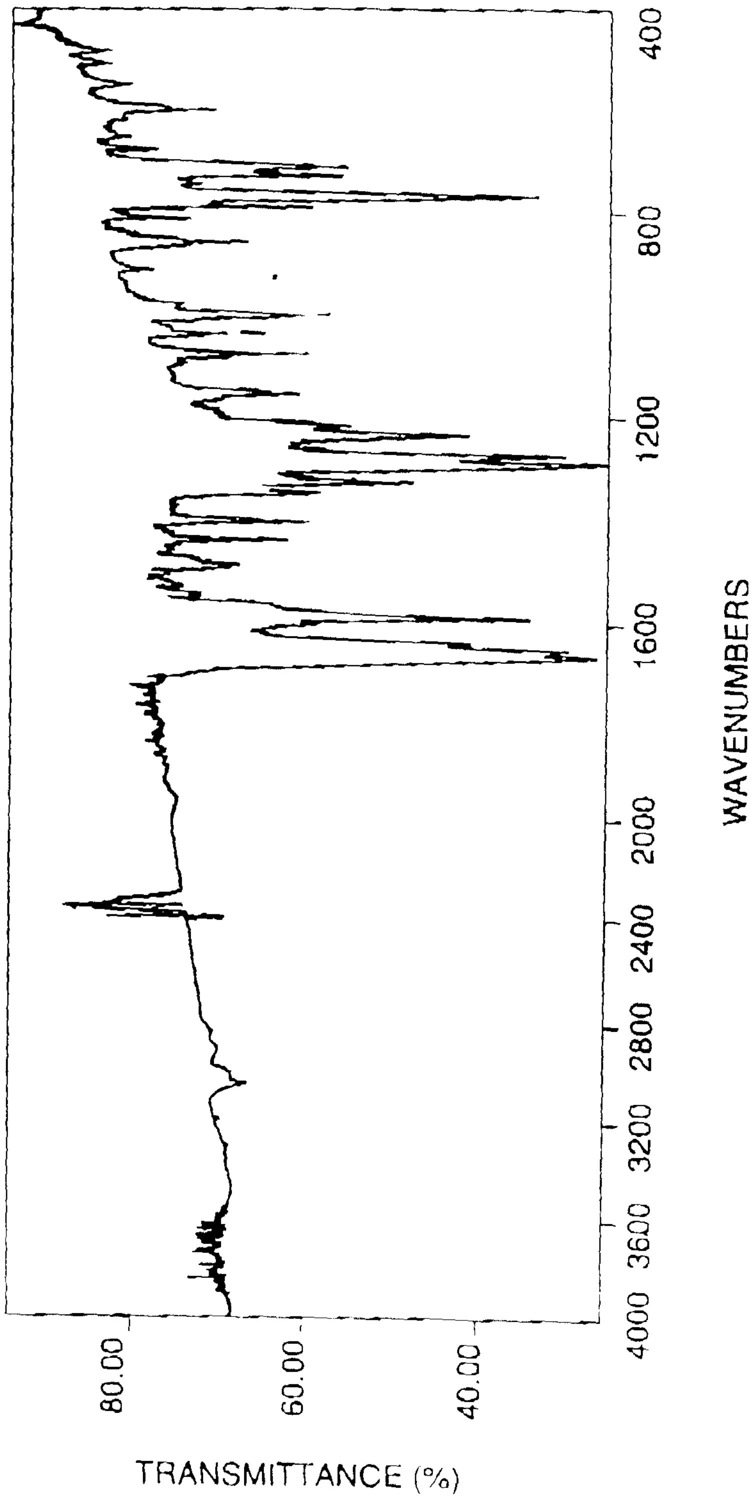
TRANSMITTANCE (%)
80.00
60.00
40.00
WAVENUMBERS
4000
3600
3200
2800
2400
2000
1600
1200
800
400

FIG.4

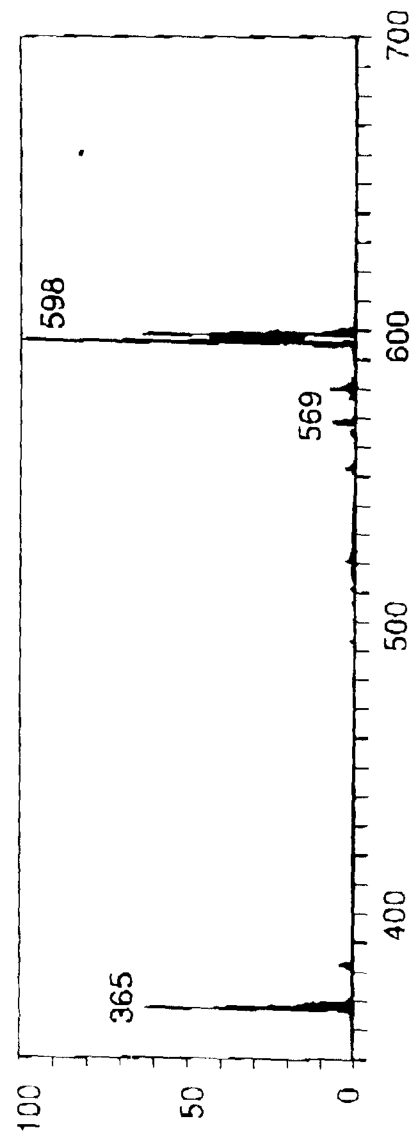

FIG.5

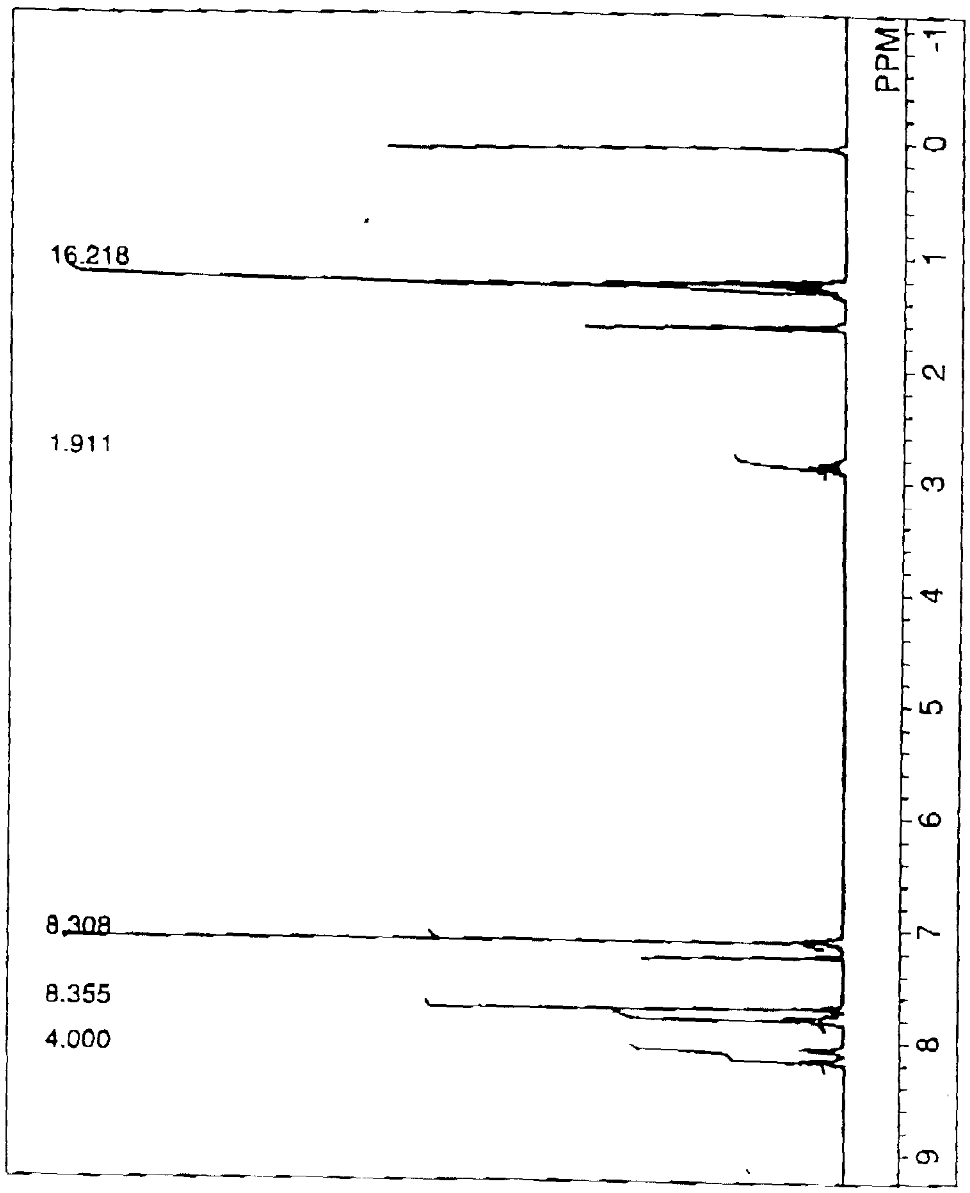

FIG.6

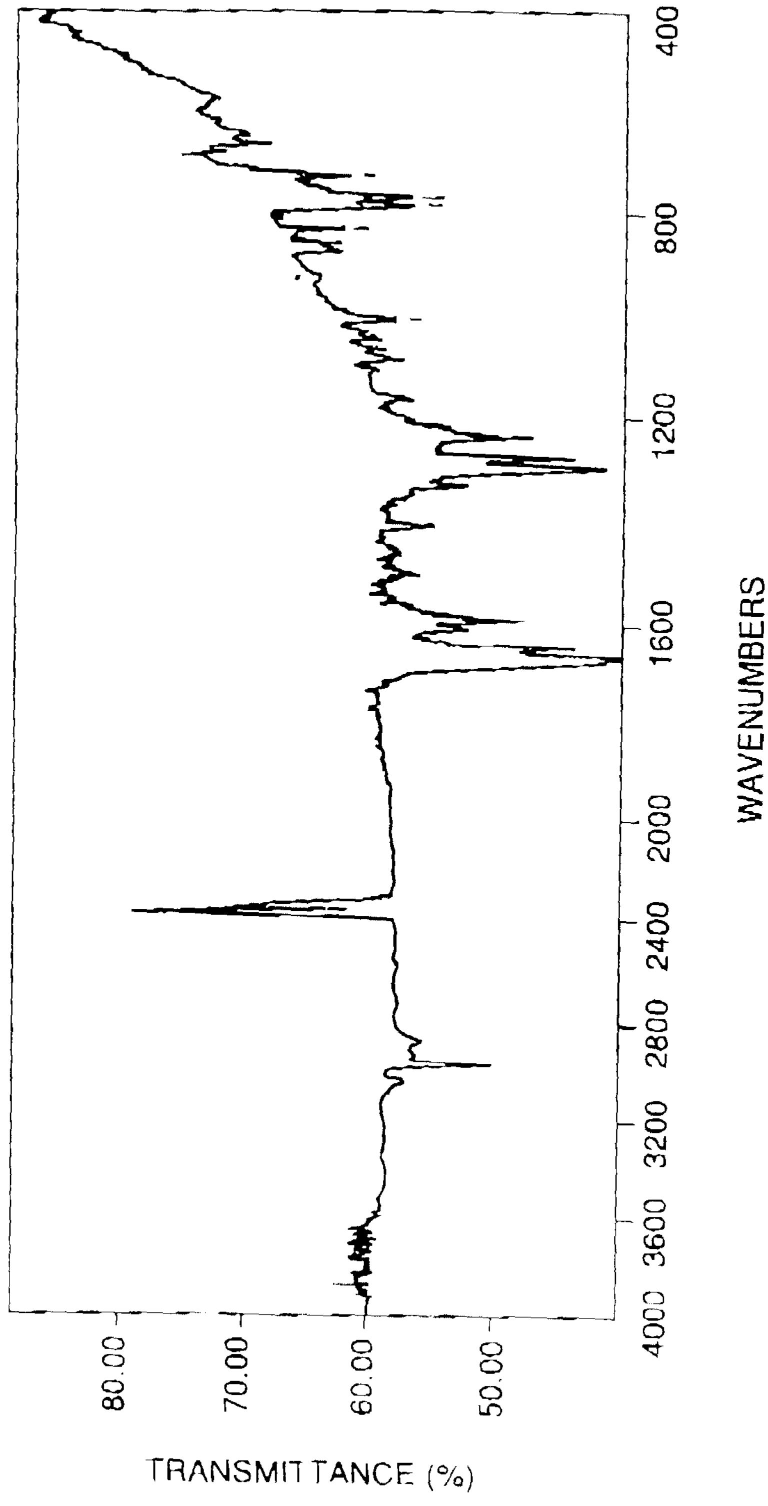
TRANSMITTANCE (%)
80.00
70.00
60.00
50.00
WAVENUMBERS
4000
3600
3200
2800
2400
2000
1600
1200
800
400

FIG.7

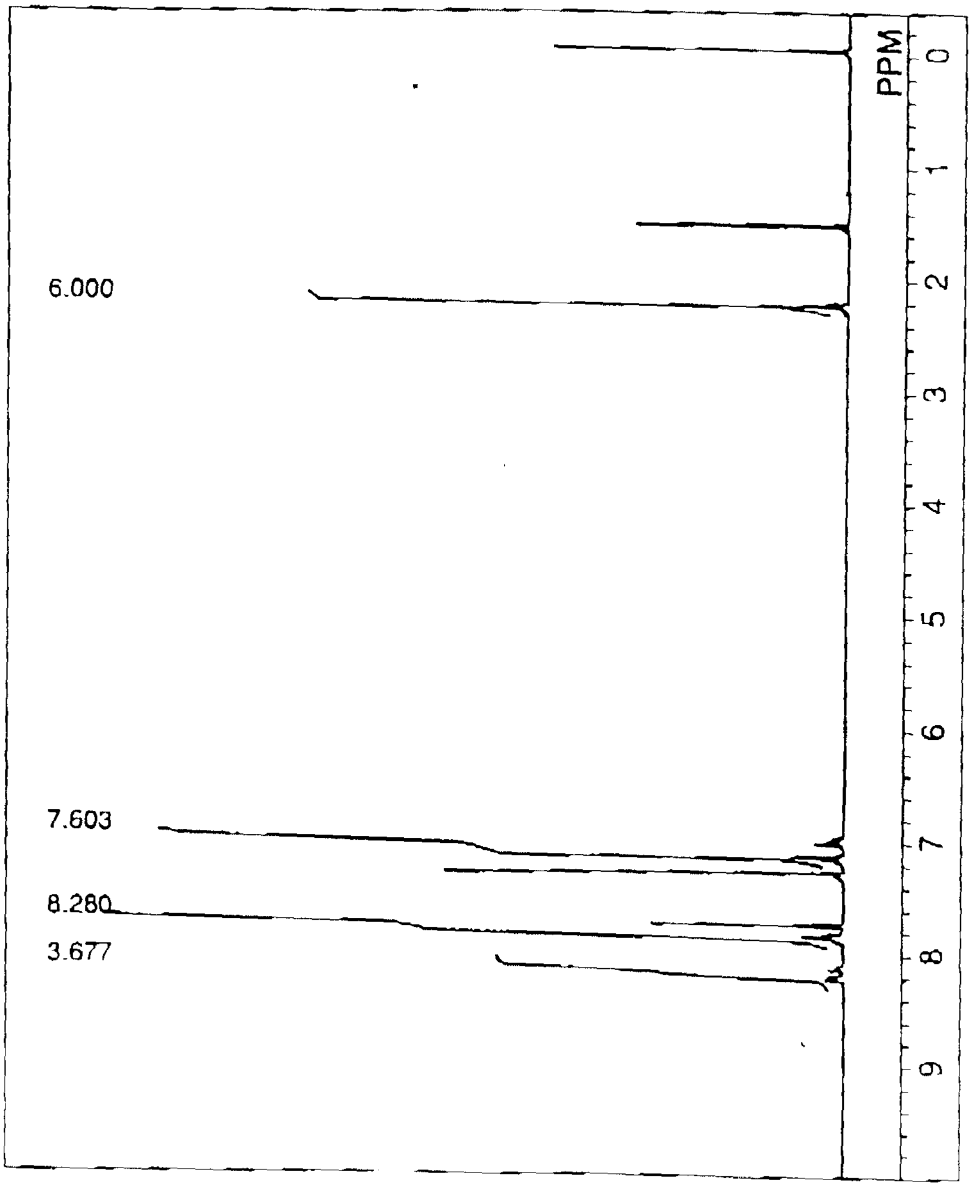

FIG.8

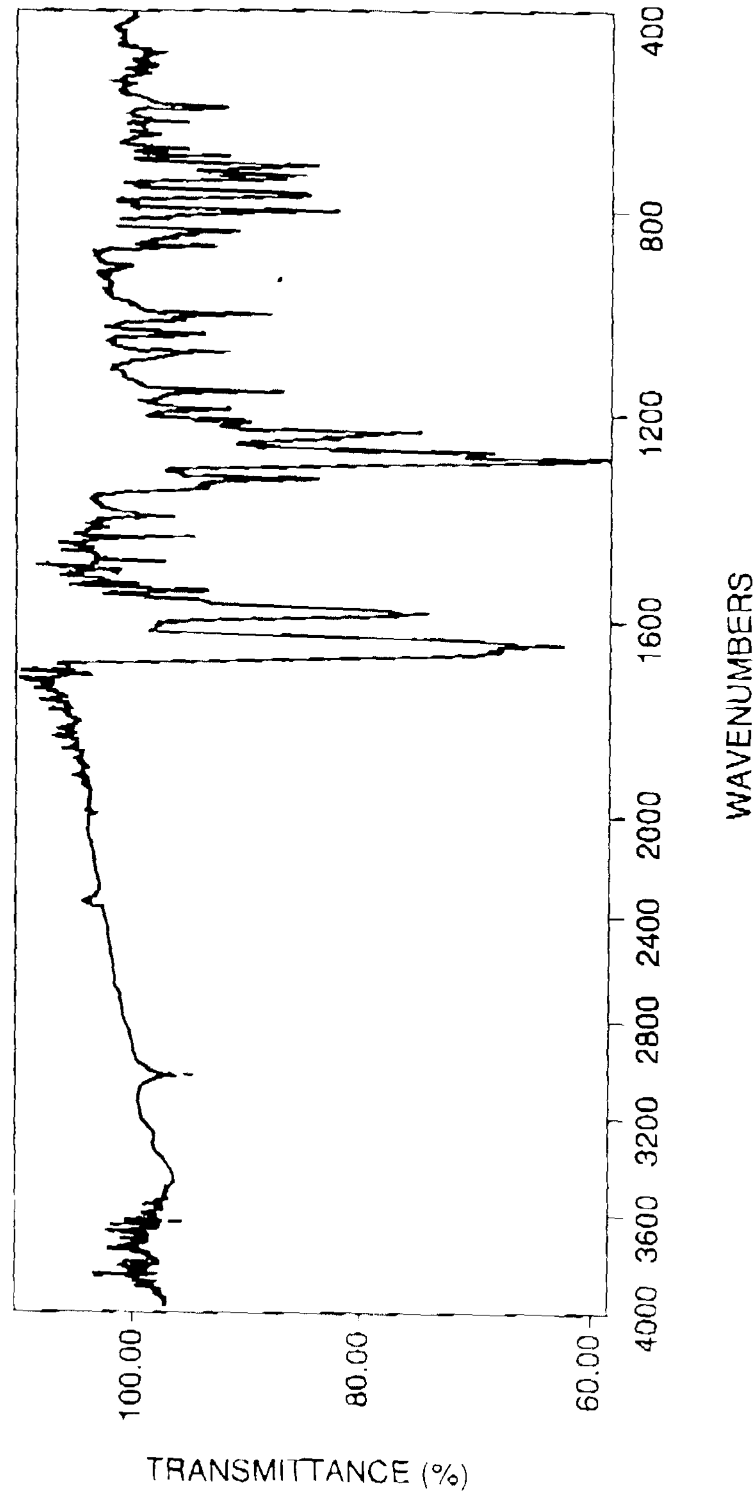

WAVENUMBERS
4000
3600
3200
2800
2400
2000
1600
1200
800
400
TRANSMITTANCE (%)
100.00
80.00
60.00

FIG.9

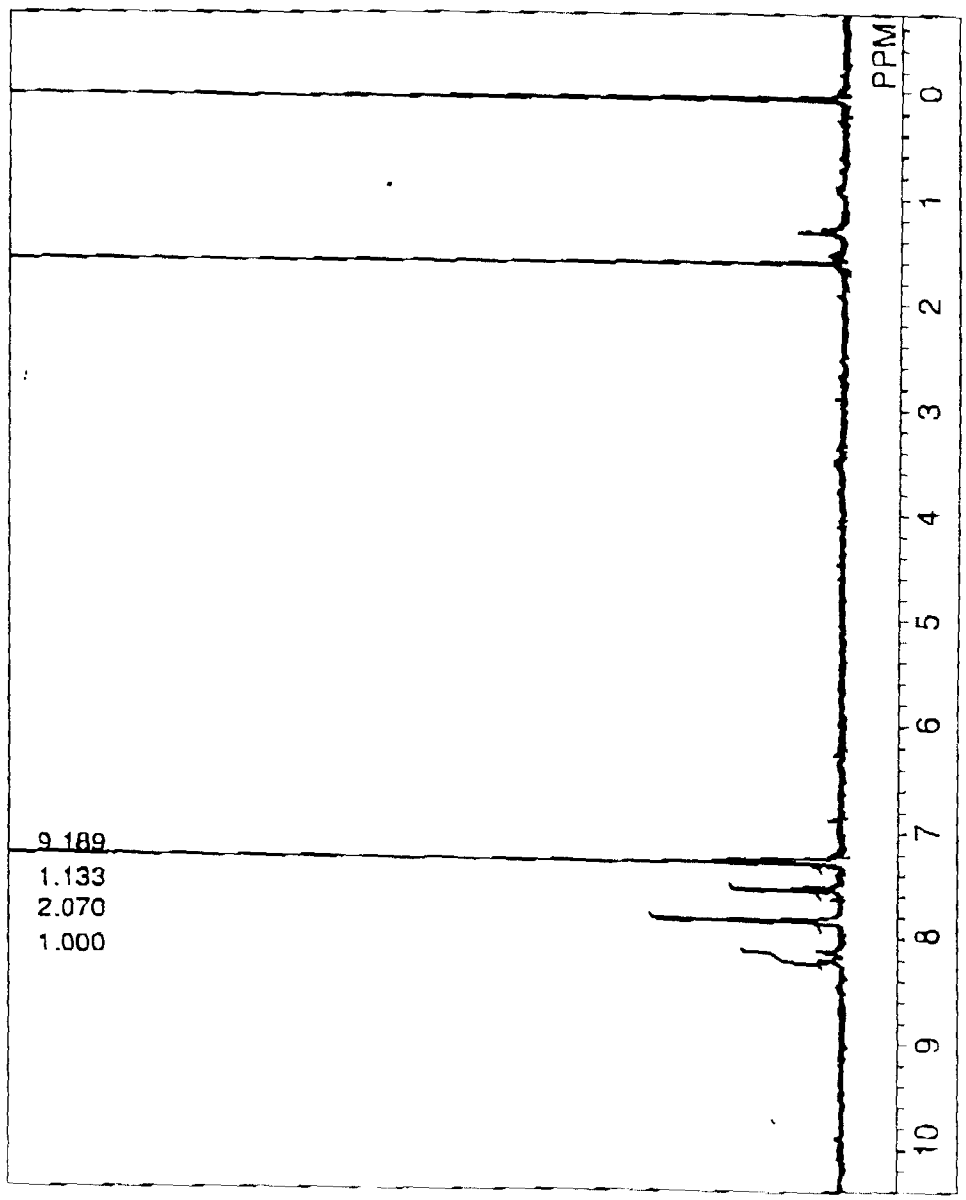

FIG.10

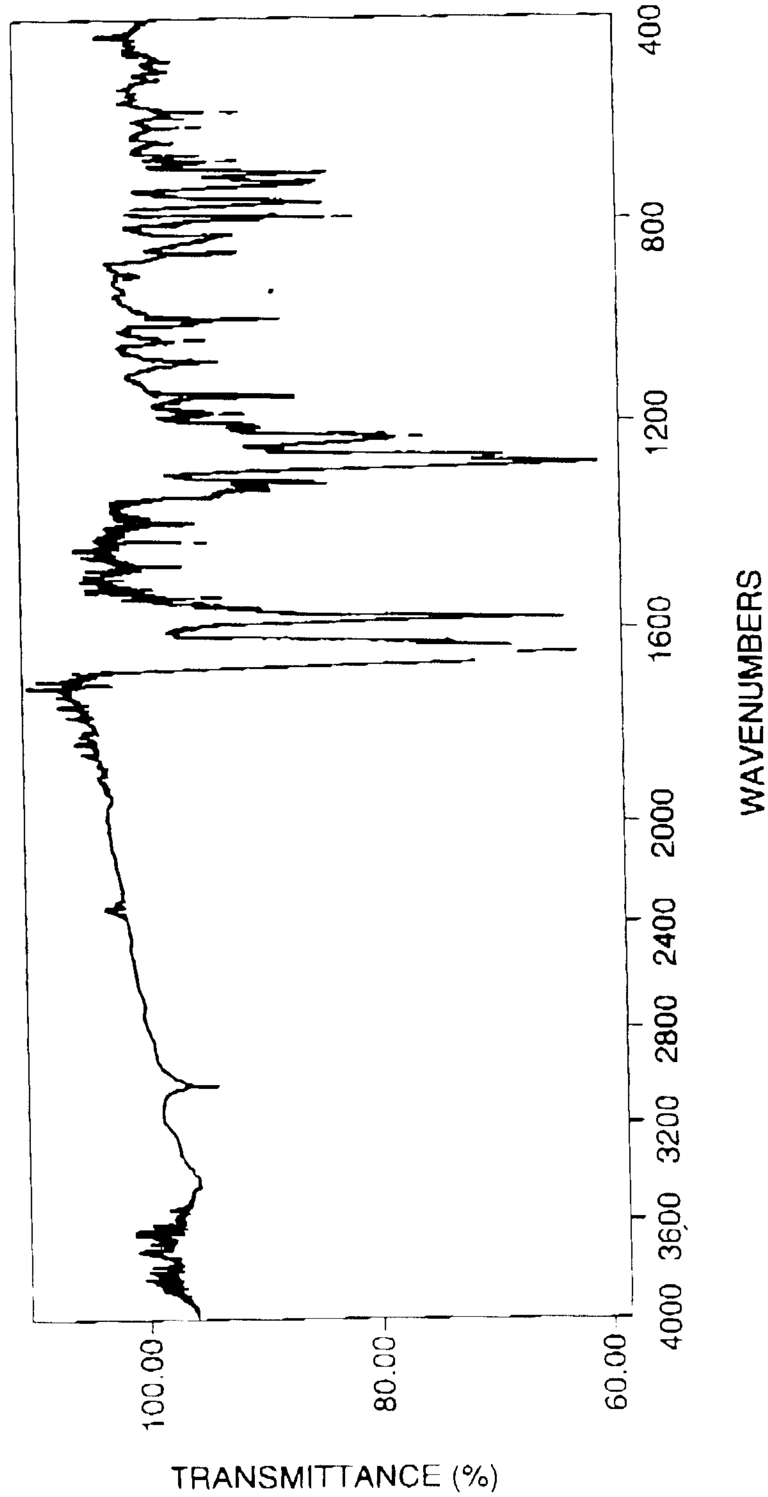

TRANSMITTANCE (%)
100.00
80.00
60.00
WAVENUMBERS
4000
3600
3200
2800
2400
2000
1600
1200
800
400

FIG.11

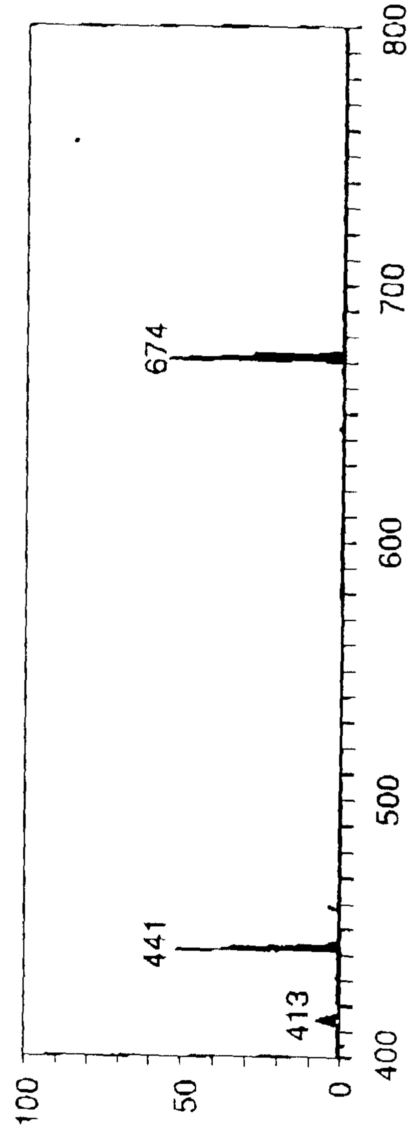

European Patent Office

# EUROPEAN SEARCH REPORT

Application Number
EP 98 30 4106

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP 0 764 886 A (MITA INDUSTRIAL CO LTD) 26 March 1997<br>* page 11 - page 12; claims *<br>--- | 1-4 | C07C50/38<br>G03G5/06 |
| A | CHEMICAL ABSTRACTS, vol. 093, no. 4, 28 July 1980<br>Columbus, Ohio, US;<br>abstract no. 035010,<br>BALABANOV E I ET AL: "Electrophotographic material"<br>XP002075322<br>* abstract *<br>& SU 720 414 - (USSR) 5 March 1980<br>--- | 1,2 | |
| A | CHEMICAL ABSTRACTS, vol. 084, no. 3, 19 January 1976<br>Columbus, Ohio, US;<br>abstract no. 017008,<br>MARKAVA E ET AL: "Isomerization of some 2,2'-(phenylene)bis(1,3-indandione) derivatives"<br>XP002075323<br>* abstract *<br>& LATV. PSR ZINAT. AKAD. VESTIS, KIM. SER. (LZAKAM);75; (5); PP.613-17,<br>Inst. Org. Sint.;Riga; USSR<br>----- | 1 | TECHNICAL FIELDS SEARCHED (Int.Cl.6)<br>C07C<br>G03G |

The present search report has been drawn up for all claims

| Place of search | Date of completion of the search | Examiner |
|---|---|---|
| THE HAGUE | 24 August 1998 | Bonnevalle, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)